# EUROPEAN PATENT APPLICATION

(11) **EP 4 509 129 A1**
(43) Date of publication of application: **19.02.2025**
(21) Application number: 23738602.4
(22) Date of filing: 14.03.2023
(51) Int. Cl.: A61K 35/747, A23L 33/135, A61P 5/30, A61P 43/00, C12N 1/20

(54) **GLUCURONIDASE ACTIVATOR, PHARMACEUTICAL COMPOSITION, EDIBLE COMPOSITION, AND COMPOSITION FOR ORAL APPLICATION**

(30) Priority: 17.03.2022 JP 2022042917
(71) Applicant: Kaneka Corporation, Osaka-shi, Osaka 530-8288 (JP)
(72) Inventor: HONDA, Shinichi, Takasago-shi, Hyogo 676-8688 (JP)
(74) Representative: Hoffmann Eitle
(86) International application number: PCT/JP2023/009929
(87) International publication number: WO 2023/176849

(57) **Abstract**

A glucuronidase activator including an active ingredient that is a *Levilactobacillus brevis* strain I-3141 deposited under accession number CECT 7480.

## Description

### Technical Field

The present invention relates to glucuronidase activators, pharmaceutical compositions, edible compositions, and oral compositions.

### Background Art

As women age, functions of ovaries decrease, which significantly reduces secretion of estrogen. In particular, a period of 10 years including 5 years before menopause and 5 years after menopause is called climacteric. In this period, various symptoms appear in bodies due to the imbalance of hormones. The symptoms occurring during climacteric are called climacteric symptoms. Among such symptoms, the condition in which the symptoms are severe and cause troubles in daily life is called climacteric syndrome. In addition, in the case of early menopause due to ovariectomy, hysterectomy, or the like, climacteric symptoms may also appear.

Regardless of its severity, climacteric symptoms cause phycological pains for women. Under such circumstances, there is a need for improving quality of life (QOL) of women by preventing or treating the climacteric symptoms.

The climacteric symptoms are mainly classified into nervous system symptoms, symptoms related to skin, mucosal membrane, or hair, sexual function symptoms, changes in body weights and metabolism, genitourinary symptoms, musculoskeletal symptoms, and the like (see Non-Patent Document 1). A major cause of climacteric symptoms is considered to be a rapid decrease of an estrogen level in a body during climacteric (see Non-Patent Document 2). Therefore, hormone replacement therapy (HRT) for supplementing deficient estrogen is mainly used as a treatment method for climacteric symptoms. However, the hormone replacement therapy has a problem such that, as side effects, a risk of occurrence of uterine bleeding, stroke, heart attack, breast cancer, uterine cancer, gastrointestinal disorder, thrombosis, hepatic disorder, or the like increases.

Therefore, there is a strong demand for a highly safe composition for preventing or treating climacteric symptoms, which is free from side effects and can prevent or treat climacteric symptoms.

Estrogen is produced in the ovary, adrenal gland, adipose tissue, other organs, and the like. Estrogen is metabolized in the liver, and part of estrogen is converted into a glucuronic acid conjugate. The glucuronic acid conjugate is secreted into the intestinal tract together with bile acids and excreted with feces. The glucuronic acid conjugate secreted in the intestinal tract is deconjugated by β-glucuronidase (GUS) activity of intestinal bacteria, and the deconjugated estrogen is reabsorbed into the body (see Non-Patent Document 3).

Healthy conditions and adequate estrogen levels are known to maintain the diversity of gut microbes. Under these conditions, beneficial bacteria predominate, which inhibit the growth of pathogenic bacteria and maintain the stability of the intestinal flora. The intestinal flora is a complex community of microorganisms that colonize the gastrointestinal tract. In bodies of postmenopausal women, conversely, estrogen deficiency alters the intestinal flora, reducing the diversity and the amount of intestinal bacteria. To combat such conditions, probiotics, which are dietary supplements or medical supplements composed of beneficial microorganisms, are known to collaborate with intestinal microorganisms to maintain the health of the host (see Non-Patent Document 4).

Lactic acid bacteria, which is one kind of intestinal bacteria, have also been used for food and drink products, such as fermented foods, pharmaceutical products, probiotics, and the like over a long period of time. Lactic acid bacteria assigned to the genus *Lacticaseibacillus* are also highly safe lactic acid bacteria used for various applications.

### Citation List

### Non-Patent Documents

Non-Patent Document 1: Patrizia Monteleone et al., Nature Reviews Endocrinology, 2018, 14(4), p. 199-215
Non-Patent Document 2: Nkechinyere Chidi-Ogbolu et al., Front Physiol, 2019, Vol. 9, Article 1834
Non-Patent Document 3: Maryann Kwa et al., J Natl Cancer Inst, 2016, 8(8)
Non-Patent Document 4: Xin Xu et al., Bone Research, 2017, 5, 17046

### Summary of Invention

### Technical Problem

The present invention aims to solve the above various problems existing in the related art, and achieve the following object. Specifically, the present invention has an object to provide a glucuronidase activator, a pharmaceutical composition, a probiotic composition, and an edible composition, which have excellent glucuronidase activity and are highly safe.

### Solution to Problem

Means for solving the above problems are as follows.
<1> A glucuronidase activator including an active ingredient that is a *Levilactobacillus brevis* strain I-3141 deposited under accession number CECT 7480.
<2> A method of enhancing glucuronidase activity including administering of a *Levilactobacillus brevis* strain I-3141 deposited under accession number CECT 7480 to a subject to enhance the glucuronidase activity.
<3> A pharmaceutical composition including the glucuronidase activator according to <1> and a pharmaceutically acceptable carrier.
<4> An edible composition including the glucuronidase activator according to <1> and an edible ingredient.
<5> An oral composition including a *Levilactobacillus brevis* strain I-3141 deposited under accession number CECT 7480, *Lactoplantibacillus plantarum* deposited under accession number CECT 7481, and *Pediococcus acidilactici* deposited under accession number CECT 7483.
<6> A method of increasing an estrogen level including orally administering of the glucuronidase activator according to <1>, the pharmaceutical composition according to <3>, the edible composition according to <4>, or the oral composition according to <5> to a subject.
<7> A method of improving estrogen metabolism including orally administering of the glucuronidase activator according to <1>, the pharmaceutical composition according to <3>, the edible composition according to <4>, or the oral composition according to <5> to a subject.
<8> A method of improving climacteric symptoms including orally administering of the glucuronidase activator according to <1>, the pharmaceutical composition according to <3>, the edible composition according to <4>, or the oral composition according to <5> to a subject.

### Effects of Invention

According to the present invention, the above various problems existing in the related art can be solved, the object can be achieved, and there are provided a glucuronidase activator, a pharmaceutical composition, a probiotic composition, and an edible composition, which have excellent glucuronidase activity and are highly safe.

### Brief Description of the Drawings

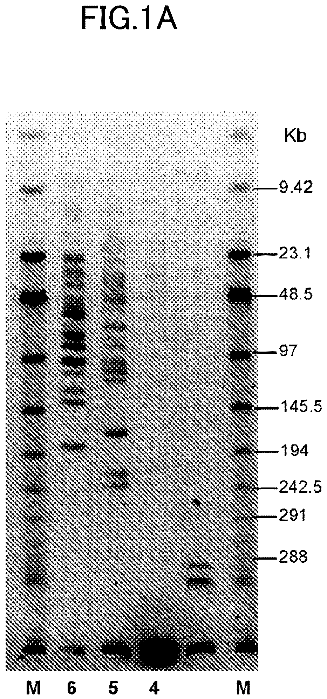

[Fig. 1A] Fig. 1A is a view depicting an electrophoresis pattern obtained by performing pulsed-field gel electrophoresis after genomic DNA digestion with a restriction enzyme Sfi-I in order to determine a genotype of a *Levilactobacillus brevis* strain I-3141. "4" indicates the *Levilactobacillus brevis* strain I-3141, "5" indicates *Lacticaseibacillus casei* VSL#3, "6" indicates *Lacticaseibacillus casei* DN114.001, and "M" indicates a molecular weight marker.

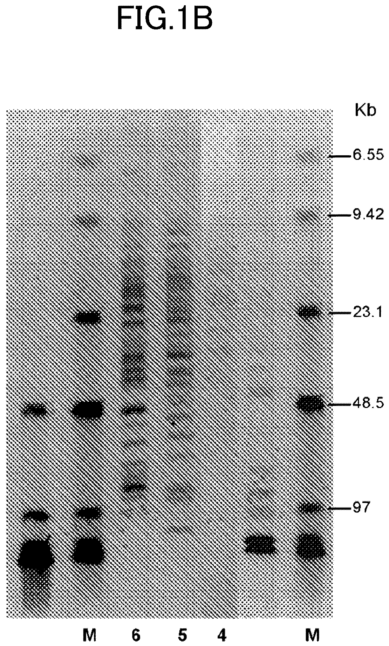

[Fig. 1B] Fig. 1B is a view depicting an electrophoresis pattern obtained by performing pulsed-field gel electrophoresis after genomic DNA digestion with a restriction enzyme Sma-I in order to determine a genotype of the *Levilactobacillus brevis* strain I-3141. "4" indicates the *Levilactobacillus brevis* strain I-3141, "5" indicates *Lacticaseibacillus casei* VSL#3, "6" indicates *Lacticaseibacillus casei* DN114.001, and "M" indicates a molecular weight marker.

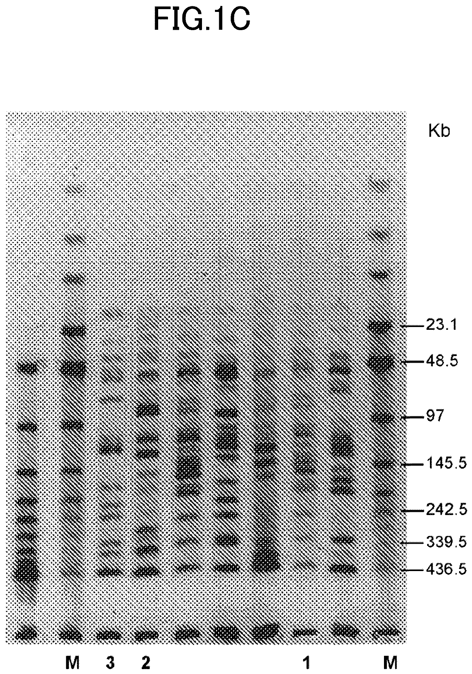

[Fig. 1C] Fig. 1C is a view depicting an electrophoresis pattern obtained by performing pulsed-field gel electrophoresis after genomic DNA digestion with a restriction enzyme Sfi-I in order to determine a genotype of the *Lactoplantibacillus plantarum* strain F-2096. "1" indicates *Lactoplantibacillus plantarum* strain F-2096, "2" indicates *Lactoplantibacillus plantarum* 299V, "3" indicates *Lacticaseibacillus casei* VSL#3, and "M" indicates a molecular weight marker.

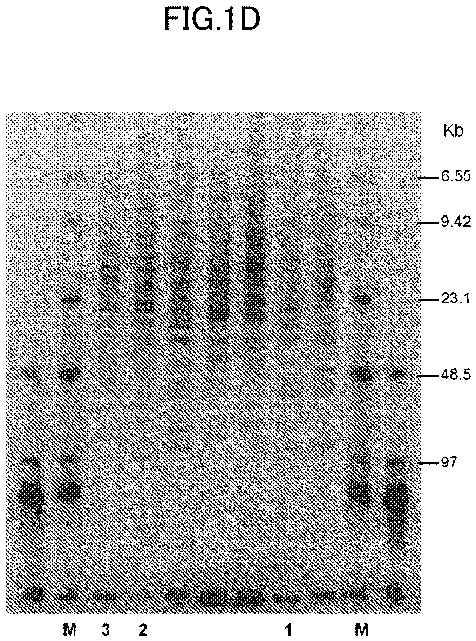

[Fig. 1D] Fig. 1D is a view depicting an electrophoresis pattern obtained by performing pulsed-field gel electrophoresis after genomic DNA digestion with a restriction enzyme Sma-I in order to determine a genotype of a *Lactoplantibacillus plantarum* strain F-2096. "1" indicates the *Lactoplantibacillus plantarum* strain F-2096, "2" indicates *Lactoplantibacillus plantarum* strain 299V, "3" indicates *Lacticaseibacillus casei* VSL#3, and "M" indicates a molecular weight marker.

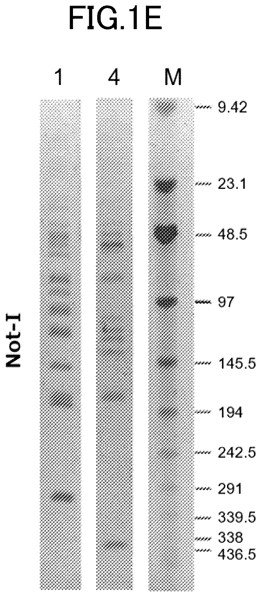

[Fig. 1E] Fig. 1E is a view depicting an electrophoresis pattern obtained by performing pulsed-field gel electrophoresis after genomic DNA digestion with a restriction enzyme Not-I in order to determine a genotype of a *Pediococcus acidilactici* strain F-1033. "1" indicates the *Pediococcus acidilactici* strain F-1033, "4" indicates *Pediococcus acidilactici* Rossell 1001, and "M" indicates a molecular weight marker.

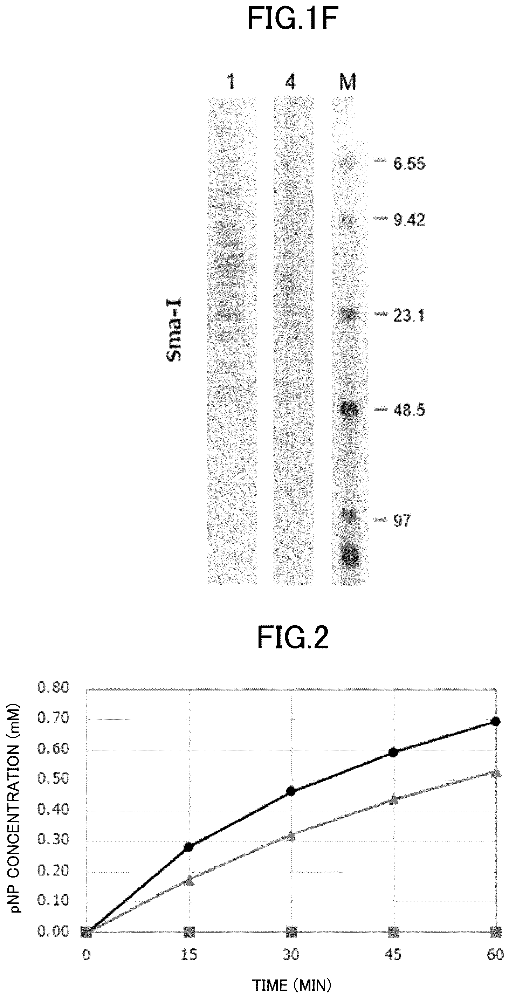

[Fig. 1F] Fig. 1F is a view depicting an electrophoresis pattern obtained by performing pulsed-field gel electrophoresis after genomic DNA digestion with a restriction enzyme Sma-I in order to determine a *Pediococcus acidilactici* strain F-1033. "1" indicates the *Pediococcus acidilactici* strain F-1033, "4" indicates *Pediococcus acidilactici* Rossell 1001, and "M" indicates a molecular weight marker.
[Fig. 2] Fig. 2 is a diagram depicting a result of GUS activity when pNGP was used as a substrate in Test Example 2. "•" indicates the result of the *Levilactobacillus brevis* strain I-3141, **"▲"** indicates the result of the *Levilactobacillus brevis* NBRC 107147, and **"■"** indicates the result of the negative control.

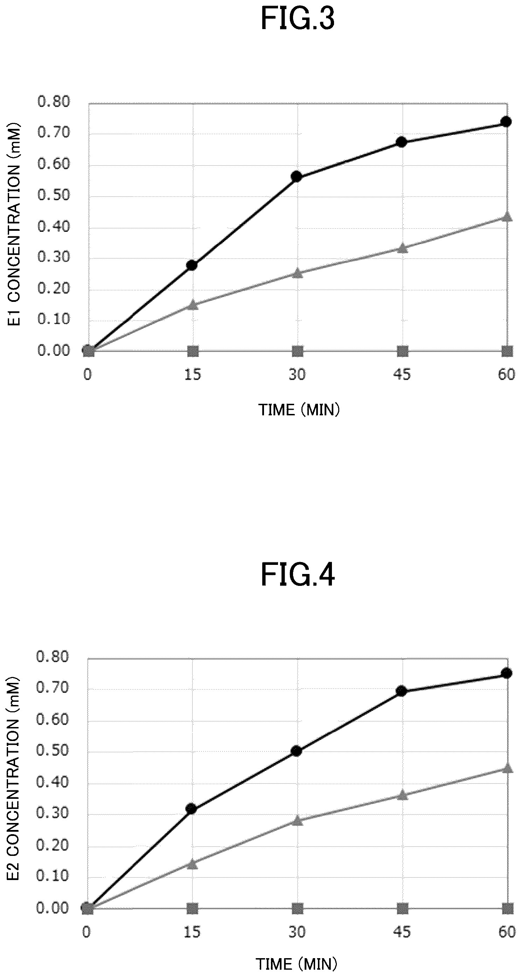

[Fig. 3] Fig. 3 is a diagram depicting a result of GUS activity when E1-3G was used as a substrate in Test Example 2. "•" represents the result of the *Levilactobacillus brevis* strain I-3141, **"▲"** represents the result of the *Levilactobacillus brevis* NBRC 107147, and **"■"** represents the result of the negative control.
[Fig. 4] Fig. 4 is a diagram depicting a result of GUS activity when E2-3G was used as a substrate in Test Example 2. "•" represents the result of the *Levilactobacillus brevis* strain I-3141, **"▲"** represents the result of the *Levilactobacillus brevis* NBRC 107147, and **"■"** represents the result of the negative control.

### Description of Embodiments

### (Microorganism)

The microorganism of the present invention is *Levilactobacillus brevis* strain I-3141 deposited under accession number CECT 7480, and has glucuronidase activity. The microorganism can deconjugate glucuronic acid conjugates of estrogen due to the glucuronidase activity.

As the glucuronidase, there are α-glucuronidase (EC3.2.1.139) and β-glucuronidase (EC3.2.1.31).

The α-glucuronidase is a hydrolase that generates alcohol and D-glucuronic acid using α-D-glucuronoside and water as substrates.

The β-glucuronidase is a collective name for enzymes that acts on β-type glucosides of D-glucuronic acid to cause hydrolysis of a glucuronide bond thereof. The β-glucuronidase has a broad specificity to aglycone and also acts on β-D-glucuronide of alcohol, steroid, carboxylic acid, and the like.

Among the above, microorganisms having β-glucuronidase activity are preferred.

A method of confirming the glucuronidase activity of the microorganism is not particularly limited, and may be appropriately selected from methods available in the related art. Examples of the method include a method in which substrates of known glucuronidase are reacted with the microorganism, and the presence or absence of a reaction product is confirmed or the concentration of the reaction product is measured, and the like.

The *Levilactobacillus brevis* strain I-3141 is as described in Japanese Patent No. 5879349 (International Patent Publication No. WO 2012/022773). An isolation method and taxonomic characteristics thereof will be described hereinafter.

### 1. Isolation of microorganism

The I-3141 strain was isolated from saliva derived from children aged 0 to 5 years old in a tropical undeveloped region of South America. The saliva was dissolved in a PBS buffer (pH 7.4), pipetted, and inoculated on MRS (Man Rogosa Sharp, produced by Sigma-Aldrich Chem, Spain) agar supplemented with 10 µg/ml vancomycin (produced by SIGMA). The bacterium was incubated at 37°C under microaerobic conditions (5% CO₂). After the growth, the isolated strain was stored by freeze-drying in 0.1×PBS including 15% by mass of skim milk powder.

### 2. Taxonomic characteristics of strain

### 2.1. Genetic identification of genera and species

### A) Method

The bacterium was incubated overnight on an MRS medium (pH 6.4) at 37°C in an atmosphere containing 5% CO₂. Further, cells of the bacterium were harvested, washed, resuspended in a lysis buffer (480 µL of 50 mM EDTA pH 8.0; 120 µL of 10 mg/mL lysozyme), and further incubated at 37°C for 60 minutes. Genomic DNA was extracted using the Wizard genomic DNA purification kit (produced by Promega). The pretreated bacterium was centrifuged at 14,000 g for 2 minutes, and the supernatant was removed, followed by carrying out the protocol of Promega. In brief, cells of the bacterium were resuspended in a nuclear lysis solution, incubated at 80°C for 5 minutes, and then cooled to room temperature. The cell lysate was incubated in a RNase solution at 37°C for 60 minutes, and proteins were precipitated by adding a protein precipitation solution and spinning at high speed. The sample was cooled, and centrifuged at 15,000 g for 3 minutes. The supernatant containing DNA was transferred into a clean 1.5 mL microcentrifuge tube and mixed with 600 µL of isopropanol by inverting. The DNA was recovered by centrifugation at 15,000 g for 2 minutes, and the supernatant was carefully discarded. The DNA sample was washed with 600 µL of 70% by volume ethanol by gently inverting the tube several times. After centrifugation at 15,000 g for 2 minutes, the ethanol was removed by suction. Finally, the DNA pellet was incubated at 65°C for 1 hour and resuspended in 100 µL of a rehydration solution. The resultant sample was stored at 2°C to 8°C.

The 16S rRNA was amplified by PCR using the universal primers Eub27f and Eub1492r, which generated fragments (1,000 or more nucleotides) of nearly the entire sequence of 16S. Next, the DNA obtained in the above-described manner was washed using QIA (registered trademark) quick kit (produced by Qiagen).

The primers used for amplifying and sequencing the 16S gene are presented in Table 1 below.

Four consecutive sequencing reactions were performed by Genetic Analyzer 3130 (produced by Applied Biosystems) using the primers presented in Table 1 below and BigDye v. 3.1 kit. Data was collected and chromatograms were generated using DNA Sequence Analysis v. 5.2 software (produced by Applied Biosystems), and the results were examined by visual analysis on Chromas (produced by Technelysium Pty Ltd.) and BioEdit (produced Ibis Biosciences).

The identification of the genus was performed using Ribosomal Database Project Tool (Wang Q et al., "Naive Bayesian Classifier for Rapid Assignment of rRNA Sequences into the New Bacteial Taxonomy", Appl Environ Microbiol, 2007, vol. 73, p. 5261-5267). The determination of the species was performed by comparing with a sequence obtained from a 16s sequence of a known organism from both RefSeq database (http://www.ncbi.nlm.nih.gov/RefSeq/) using BLASTN, and Ribosomal Database roject (http://rdp.cme.msu.edu/, J.R. Cole et al., "The Ribosomal Database Project (RDP-II): introducing myRDP space and quality controlled public data", Nucl. Acids Res., 2007, vol. 35, p. 169-172).

**[Table 1]**

| PROCESS | PRIMER | DIRECTION | 5' →3' SEQUENCE |
|---|---|---|---|
| AMPLIFICATION | Eub27f | FORWARD | GAGTTTGATCCTGGCTCAG (SEQ ID NO: 1) |
| | Eub1492r | REVERSE | TACGGYTACCTTOTTACGACTT (SEQ ID NO: 2) |
| SEQUENCING | 27f | FORWARD | AGAGTTTGATCCTGGCTCAG (SEQ ID NO: 3) |
| | 357f | FORWARD | CGCCCGCCGCGCCCCGCGCCCGGCCCGC CGCCCCCGCCCCCCTACGGGAGGCAGCAG (SEQ ID NO: 4) |
| | 907r | REVERSE | CCGTCAATTCCTTTGAGTTT (SEQ ID NO: 5) |
| | 1492r | REVERSE | GGTTACCTTGTTACGACTT (SEQ ID NO: 6) |

### B) Result

The strain I-3141 was determined as being assigned to the *Levilactobacillus brevis* species by the RDP (Ribosimal Database Project) tool.

### 2.2. Determination of genotype of strain

### A) Method

Characterization was performed by genomic DNA digestion and pulsed-field gel electrophoresis. The strain I-3141 was subjected to the previously described protocol (Rodas AM et al., "Polyphasic study of wine Lactobacillus strains: taxonomic implications", Int J Syst Evol Microbiol, 2005, vol. 55, p. 197-207*). Lacticaseibacillus casei* VSL#3 and *Lacticaseibacillus casei* DN114.001, which were commercially available strains, were included in an assay as control strains. All strains were grown on an MRS agar plate, and incubated at 37°C for 18 hours in 5% CO₂. The cells were harvested and washed three times in 8 mL of PET (10 mM Tris pH 7.6, 1 M NaCl), followed by centrifugation at 6,000 rpm for 10 minutes. The pellet was resuspended in 700 µL of a lysis buffer (6 mM Tris, 1 M NaCl, 0.1 M EDTA, 0.5% SLS, 0.2% deoxycholic acid; 1 mg/mL lysozyme; 40 U/mL mutanolysin; 20 g/mL RNase). An equal volume of 1.6% low-melting-point agarose (FMC BioProducts, Rockland, ME, USA) was added to the resuspended cells and allowed to solidify at 4°C for 1 hour. The inserted product was transferred to 2 mL of a lysis buffer II (0.5 M EDTA pH 9.2, 1% N-laurylsarcosine, and 1 mg/mL pronase), and incubated at 50°C for 48 hours. Next, the inserted product was washed with a TE buffer (10 mM Tris, 1 mM EDTA pH 8.0) at room temperature. Digestion of a whole DNA was performed with a restriction enzyme Sfi-I or restriction enzyme Sma-I (both produced by Roche Diagnostics).

The pulsed-field electrophoresis was performed by a CHEF DR III system (BioRad Laboratories). The inserted product loaded onto a 1% agarose gel (SeaKem ME agarose, FMC BioProducts, ME, USA). Table 2 below describes the electrophoretic conditions for Sfi-I or Sma-I restricted genomic DNA derived from the strain I-3141. The DNA molecular weight markers were Lambda ladder PFG Marker and Low Range PFG Marker (both produced by New England Biolabs). After the electrophoresis, the gel was stained with ethidium bromide and UV using the GelDoc System (produced by BioRad) .

Note that, the *Lacticaseibacillus casei* VSL#3 used as a control strain was isolated from VSL#3 (registered trademark) (produced by Alfasigma USA, Inc.) on an MRS agar plate. Moreover, *Lacticaseibacillus casei* DN114.001 was isolated from Actimel (registered trademark) (produced by Danone) on an agar plate. For both control strains, cells incubated at 37°C under 5% CO₂ were used.

**[Table 2]**

| ENZYME | BLOCK | FIRST PULSE (SECOND) | LAST PULSE (SECOND) | TIME (HOUR) |
|---|---|---|---|---|
| Sfi-I | 1 | 2 | 10 | 10 |
| | 2 | 15 | 25 | 6 |
| Sma-I | 1 | 0.5 | 5 | 16 |

### B) Result

As depicted in Figs. 1A and 1B, the cleavage pattern obtained by the Sfi-I or Sma-I digestion of the strain I-3141 is different from those of the commercially available *Lacticaseibacillus casei* strains that are very closely related to the strain I-3141.

The strain I-3141 is considered as being assigned to *Levilactobacillus brevis* from the results above. Therefore, the strain I-3141 was determined as a *Levilactobacillus brevis* strain I-3141 (may be merely referred to as a "strain I-3141" hereinafter).

Note that, the strain I-3141 was deposited at the Spanish Type Culture Collection (COLECCION ESPANOLA DE CULTIVOS TIPO (CECT)) (location: Edificio 3 CUE. Parc Cientific Universitat de Valencia Catedratico Agustin Escardino, 9 46980 Paterna(Valencia) Spain) by a depositor, AB-BIOTICS S.A. (residence: Av. Torre Blanca 57 ESADE Creapolis Building Sant Cugat del Valles 08172 Spain), and was internationally deposited on January 29, 2013 under accession number CECT 7480.

Since it had been reported that bacteria assigned to the genus *Lactobacillus* are systematically diverse and physiological and biochemical characteristics thereof were greatly different among bacterial species, the genus classification was performed at the genome level in 2020 (see Zheng J et al., Int J Syst Evol Microbiol, 2020, Apr; 70(4), p. 2782-2858).

The *Levilactobacillus brevis* to which the strain I-3141 is assigned corresponds to *Lactobacillus brevis* classified according to the classification before the genus classification in 2020.

As seen in other bacteria, the strain I-3141 may have altered properties. The microorganism of the present invention includes the strain I-3141 having altered properties as long as the strain I-3141 has the glucuronidase activity.

Examples of the strain in which the properties of the strain I-3141 are altered include mutant strains derived from the strain I-3141 (e.g., natural mutant strains, and artificial mutant strains obtained by a mutation treatment with ultraviolet light, X-rays, radiation, chemicals, etc.), genetically modified products (e.g., transformants), and the like.

Examples of a method of incubating the microorganism include a method in which the microorganism is added to a nutrient medium (may be merely referred to as a "medium" hereinafter) to incubate the microorganism at a temperature at which the microorganism can grow.

The nutrient medium is not particularly limited, and may be appropriately selected according to the intended purpose. For example, nutrient media, which have been used for incubating lactic acid bacteria and are known in the related art, may be used.

A nutrient source to be added to the nutrient medium is not particularly limited, and may be appropriately selected according to the intended purpose.

As a carbon source, for example, organic carbon compounds, such as D-ribose, D-galactose, D-glucose, D-fructose, D-mannose, D-mannitol, N-acetylglucosamine, amygdalin, arbutin, esculin, salicin, D-cellobiose, D-maltose, sucrose, D-trehalose, gentiobiose, molasses, starch syrup, oil and fat, and the like can be used.

As a nitrogen source, for example, organic or inorganic nitrogen compounds, such as meat extracts, casein, peptone, yeast extracts, dry yeast, embryo, soybean powder, urea, amino acids, peptides, corn steep liquor, ammonium salts and the like can be used.

Moreover, inorganic salts, such as sodium salts, potassium salts, calcium salts, magnesium salts, phosphoric acid salts, iron salts, copper salts, zinc salts, cobalt salt, and the like can be also used.

Furthermore, organic acids, such as citric acid and the like, vitamins, such as biotin, vitamin B1, and the like, cystine, surfactants, growth promotors, such as methyl oleate, lard oil, and the like may be used.

The above materials of the nutrient medium may be any materials with which the strain I-3141 can be used, and all culture materials known in the related art can be used.

The pH of the medium is not particularly limited as long as growth of the microorganism is not substantially inhibited. The pH of the medium is preferably 3.0 to 8.0.

Examples of the prepared nutrient medium include MRS media, GAM media, Rogosa media, and the like.

Conditions of incubating the microorganism are not particularly limited. The incubation can be performed according to typical incubation conditions for microorganisms of the genus *Lactobacillus.* In the case of a liquid culture method, static culture is preferred. A scale of the incubation is not particularly limited, and the incubation can be performed in a test tube, flask, a fermenter, or the like.

A temperature of incubating the microorganism is not particularly limited as long as the temperature is within a range in which growth of the microorganism is not substantially inhibited. The temperature of incubating the microorganism is preferably 15°C to 45°C, and more preferably 30°C to 40°C.

An incubation time is not particularly limited, and may be appropriately selected according to the intended purpose.

As glucuronic acid conjugates of estrogen present in a body, estrone 3-(β-D-glucuronide) (E1-3G) represented by the following structural formula (1), estradiol 3-(β-D-glucuronide) (E2-3G) represented by the following structural formula (2), estradiol 17-(β-D-glucuronide) (E2-17G) represented by the following structural formula (3), estriol 3-(β-D-glucuronide) (E3-3G) represented by the following structural formula (4), estriol 16-(β-D-glucuronide) (E3-16G) represented by the following structural formula (5), estriol 17-(β-D-glucuronide) (E3-17G) represented by the following structural formula (6), and the like are known.

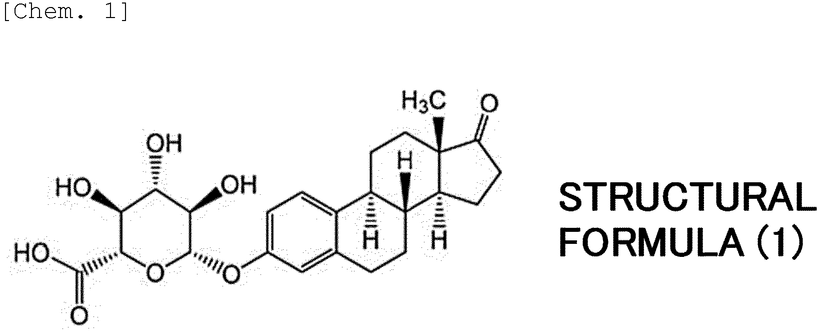

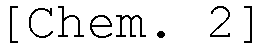

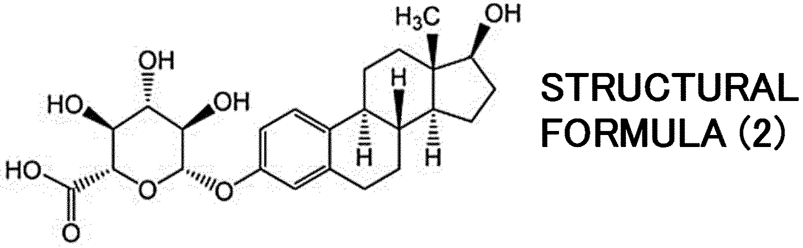

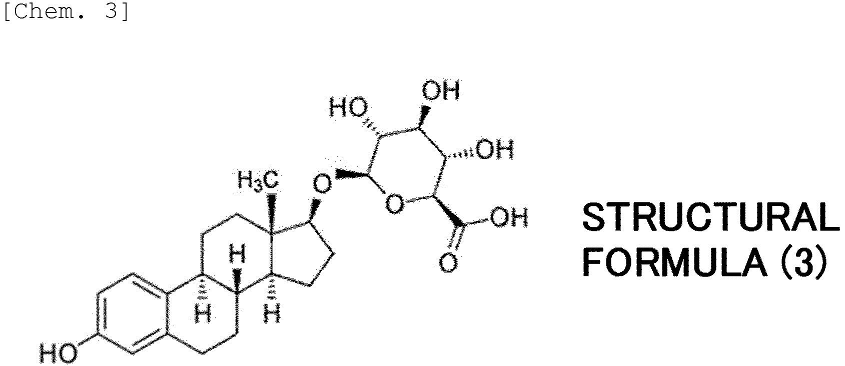

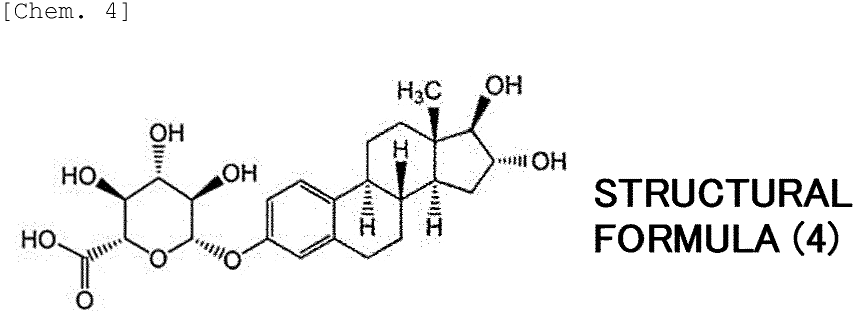

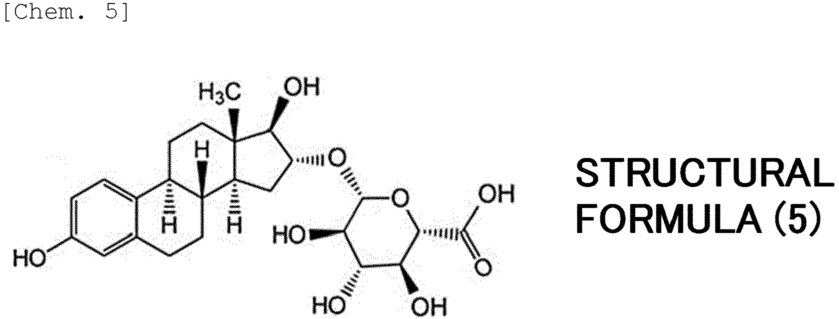

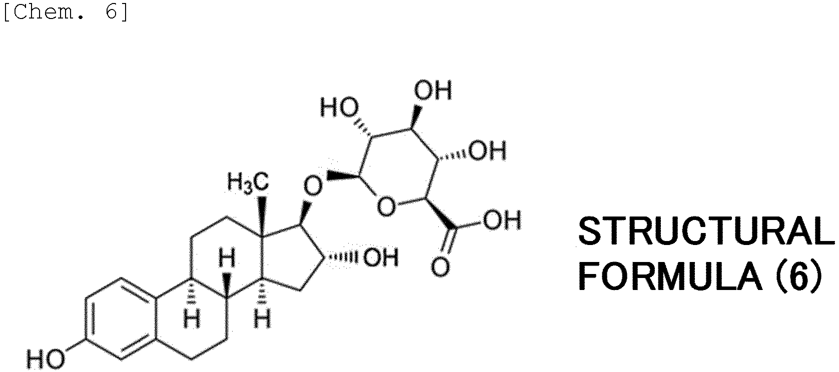

There are 3 types of the estrogen, which are estrone (E1), estradiol (E2), and estriol (E3). The major estrogen produced before menopause is estradiol (E2).

The E1-3G is deconjugated to be estrone (E1), the E2-3G or the E2-17G is deconjugated to be estradiol (E2), and the E3-3G, the E3-16G, or the E3-17G is deconjugated to be estriol (E3) (see Samantha M. Ervin et al., J Biol Chem, 2019 Dec 6, 294(49), p. 18586-18599).

The bacterium can deconjugate all of the E1-3G, the E2-3G, the E2-17G, the E3-3G, the E3-16G, and the E-17G, but preferably deconjugates a glucuronic acid conjugate E2-3G.

### -Use-

The microorganism has glucuronidase activity, and is highly safe. Therefore, the microorganism can be suitably used for increasing an estrogen level in a body, improving estrogen metabolism in a body, improving climacteric symptoms, or enhancing glucuronidase activity in an intestinal tract. Moreover, the microorganism is also suitably used for the below-described glucuronidase activator, pharmaceutical composition, probiotic composition, edible composition, oral composition, and microorganism-containing composition. Moreover, the microorganism is also suitably used in a method of enhancing glucuronidase activity, where the microorganism is administered to a subject to enhance glucuronidase activity. All of the above are included the scope of the present invention.

In the present specification, "increase of an estrogen level" means that a concentration of estrogen in a body is increased after administration of the glucuronidase activator or in a group with administration of the glucuronidase activator as compared with a concentration of estrogen in a body before administration of the glucuronidase activator or in a group without administration of the glucuronidase activator.

A method of measuring an estrogen level in a body is not particularly limited, and may be appropriately selected from methods available in the related art. Examples of the method include a method of measuring a concentration of estrogen in blood through enzyme immunoassay, chemiluminescence immunoassay, chemiluminescence enzyme immunoassay, liquid chromatography, gas chromatography, liquid chromatography mass spectrometry, or the like.

In the present specification, moreover, "improvement of estrogen metabolism" means that the estrogen level or estrogen metabolism in a body is brought closer to a healthy state, or returned to a healthy state due to the glucuronidase activity of the *Levilactobacillus brevis* strain I-3141 included in the glucuronidase activator.

In the present specification, the "healthy state" means that estrogen metabolism is a same state as the state before menopause, or a state in which no climacteric symptoms are not observed.

In a method using the microorganism as a glucuronidase activator, the microorganism may be used in combination with a below-described lactic acid bacterium assigned to *Lactoplantibacillus plantarum,* lactic acid bacterium assigned to *Pediococcus acidilactici,* or both. In view of enhancement of glucuronidase activity, three kinds of lactic acid bacteria, which are the microorganism (I-3141 strain), a lactic acid bacterium assigned to *Lactoplantibacillus plantarum,* and a lactic acid bacterium assigned to *Pediococcus acidilactici,* are preferably used, and three kinds of lactic acid bacteria, which are the microorganism (I-3141 strain), *Lactoplantibacillus plantarum* deposited under accession number CECT 7481, and *Pediococcus acidilactici* deposited under accession number CECT 7483, are more preferably used.

### (Glucuronidase activator)

The glucuronidase activator of the present invention includes a *Levilactobacillus* brevis strain I-3141 deposited under accession number CECT 7480 as an active ingredient, and may further include other ingredients as necessary.

Glucuronic acid conjugates of estrogen secreted in an intestinal tract are generally deconjugated to be estrogen due to a β-glucuronidase of intestinal bacteria, and the estrogen is then reabsorbed through the intestinal tract. However, an estrogen level in a body decreases with menopause or aging.

The strain I-3141 included in the glucuronidase activator has glucuronidase activity, thus can deconjugate a glucuronic acid conjugate of estrogen and is highly safe. Therefore, for example, the intake of the glucuronidase activator is advantageous because the glucuronidase activator can facilitate deconjugation of glucuronic acid conjugates of estrogen secreted in an intestinal tract, leading to an increase in an estrogen level in a body.

### <Strain I-3141>

The *Levilactobacillus brevis* strain I-3141 deposited under accession number CECT 7480 included in the glucuronidase activator is as described in the above section of (Microorganism).

A state of the strain I-3141 included in the glucuronidase activator is not particularly limited, and may be appropriately selected according to the intended purpose. The state thereof is preferably living bacterial cells of the strain I-3141 or a fragmented product of bacterial cells of the strain I-3141.

The living bacterial cells may be wet bacterial cells or dried bacterial cells. Specific examples of the living bacterial cells include a culture of the strain I-3141, a concentrate of the culture, a dried product of the culture, a diluted product of the culture, and the like.

A method of drying the culture is not particularly limited as long as effects obtainable by the present invention are not adversely affected, and may be appropriately selected from methods known in the related art. Examples of the method include spray drying, freeze drying, vacuum drying, drum drying, and the like.

A disrupted product of the bacterial cells refers to a state where there is no undisrupted bacterial cells as observed under a microscope.

A method of obtaining the disrupted product of the bacterial cells is not particularly limited as long as effects obtainable by the present invention are not adversely affected, and may be appropriately selected from methods known in the related art. Examples of the method include bead disruption, pressure disruption, ultrasonic disruption, rotary blade disruption, and the like.

An amount of the strain I-3141 in the glucuronidase activator is not particularly limited as long as glucuronidase activity is exhibited, and may be appropriately selected according to the intended purpose. Note that, the glucuronidase activator may be the strain I-3141 itself.

### <Other ingredients>

The above other ingredients in the glucuronidase activator are not particularly limited as long as effects obtainable by the present invention are not adversely affected, and ingredients usable for pharmaceutical products, food products, cosmetic products, and the like available in the related art may be appropriately selected according to an intended dosage form or the like. Examples of the above other ingredients include excipients, binders, disintegrants, lubricants, flavoring agents, odor correctives, moisture-proof agents, preservatives, enhancers, thickeners, emulsifiers, antioxidants, sweeteners, acidulants, seasonings, colorants, flavors, oily components, ultraviolet absorbers, surfactants, thickeners, alcohols, powder components, pigments, aqueous components,, water, oil and fat, lactic acid bacteria other than the strain I-3141, and the like. The above ingredients may be used alone or in combination.

An amount of the above other ingredients in the glucuronidase activator is not particularly limited as long as effects obtainable by the present invention are not adversely affected, and may be appropriately selected according to the intended purpose.

### <<Lactic acid bacteria other than strain I-3141>>

The lactic acid bacteria other than the strain I-3141 are not particularly limited as long as effects obtainable by the present invention are not adversely affected, and may be appropriately selected from lactic acid bacteria known in the related art. In view of increase in an estrogen level in a body, improvement of estrogen metabolism in a body, or improvement of climacteric symptoms, a lactic acid bacterium assigned to *Lactoplantibacillus plantarum* and a lactic acid bacterium assigned to *Pediococcus acidilactici* are preferred. The above lactic acid bacteria may be used alone or in combination.

### -Lactic acid bacterium assigned to Lactoplantibacillus plantarum-

The lactic acid bacterium assigned to the *Lactoplantibacillus plantarum* is not particularly limited as long as effects obtainable by the present invention are not adversely affected. The lactic acid bacterium assigned to *Lactoplantibacillus plantarum* is preferably *Lactoplantibacillus plantarum* strain F-2096 deposited under accession number CECT 7481.

The *Lactoplantibacillus plantarum* strain F-2096 is as described in International Patent Publication No. WO 2012/022773. An isolation method and taxonomic characteristics will be described hereinafter.

Similarly to the strain I-3141, the strain F-2096 is a strain isolated from saliva derived from children 0 to 5 years old in a tropical undeveloped region of South America, is isolated by the method described in "1. Isolation of microorganism" of the strain I-3141, and is determined as being assigned to the *Pediococcus acidilactici* species by the method described in "A) Method" of **"2.** Taxonomic characteristics of strain" of the strain I-3141.

Moreover, a genotype of the strain F-2096 was determined in the following manner.

A genotype of the strain F-2096 was determined in the same manner as in "A) Method" of **"2.2.** Determination of genotype of strain" of the strain I-3141, except that the commercially available strains of *Lacticaseibacillus casei* VSL#3 and *Lacticaseibacillus casei* DN114.001 included in an assay as control strains were changed to commercially available strains of *Lactoplantibacillus plantarum* 299V and *Lacticaseibacillus casei* VSL#3 included in an assay as control strains.

Note that, the *Lactoplantibacillus plantarum* 299V used as the control strain was a strain that was isolated from Ideal Bowel Support (registered trademark) (produced by Jarrow Formula) on an MRS agar plate and incubated at 37°C in 5% CO₂.

As a result, as depicted in Figs. 1C and 1D, the cleavage pattern obtained by the Sfi-I or Sma-I digestion of the strain F-2096 was different from those of the commercially available *Lactoplantibacillus plantarum* 299V and *Lacticaseibacillus casei* VSL#3 that were very closely related to the strain F-2096.

The strain F-2096 is considered as being assigned to *Lactoplantibacillus plantarum* from the results above. Therefore, the strain was determined as a *Lactoplantibacillus plantarum* strain F-2096 (may be merely referred to as a "strain F-2096" hereinafter).

Note that, the strain F-2096 was deposited at the Spanish Type Culture Collection (COLECCION ESPANOLA DE CULTIVOS TIPO (CECT)) (location: Edificio 3 CUE. Parc Cientific Universitat de Valencia Catedratico Agustin Escardino, 9 46980 Paterna(Valencia) Spain) by a depositor, AB-BIOTICS **S.A.** (residence: Av. Torre Blanca 57 ESADE Creapolis Building Sant Cugat del Valles 08172 Spain), and was internationally deposited on January 29, 2013 under accession number CECT 7481.

Since it had been reported that bacteria assigned to the genus *Lactoplantibacillusthe* are systematically diverse and physiological and biochemical characteristics thereof were greatly different among bacterial species, the genus classification was performed at the genome level in 2020 (see Zheng J et al., Int J Syst Evol Microbiol, 2020, Apr; 70(4), p. 2782-2858).

The *Lactoplantibacillus plantarum* to which the strain F-2096 is assigned corresponds to *Lactobacillus plantarum* classified according to the classification before the genus classification in 2020.

As seen in other bacteria, the strain F-2096 may have altered properties. The glucuronidase activator can include the strain F-2096 having altered properties as long as related characteristics and advantages of the glucuronidase activator described in the present specification can be retained or enhanced, and effects obtainable by the present invention are not adversely affected.

Examples of the strain F-2096 having altered properties include mutant strains derived from the strain F-2096 (e.g., natural mutant strains, and artificial mutant strains obtained by a mutation treatment with ultraviolet light, X-rays, radiation, chemicals, etc.), genetically modified products (e.g., transformants), and the like.

As a method of incubating the lactic acid bacterium assigned to the *Lactoplantibacillus plantarum,* the lactic acid bacterium can be incubated in the same method as the method of culturing the microorganism of the present invention, and the same medium and the like can be also used.

A state of the lactic acid bacterium assigned to the *Lactoplantibacillus plantarum* included in the glucuronidase activator is not particularly limited, and may be appropriately selected according to the intended purpose. As the state of the lactic acid bacterium assigned to the *Lactoplantibacillus plantarum,* living bacterial cells of the lactic acid bacterium assigned to the *Lactoplantibacillus plantarum* or a disrupted product of the bacterial cells of the lactic acid bacterium assigned to the *Lactoplantibacillus plantarum* is preferred.

The living bacterial cells may be wet bacterial cells or dried bacterial cells. Specific examples of the living bacterial cells include a culture of the lactic acid bacterium assigned to the *Lactoplantibacillus plantarum,* a concentrate of the culture, a dried product of the culture, a diluted product of the culture, and the like.

### -Lactic acid bacterium assigned to Pediococcus acidilactici-

The lactic acid bacterium assigned to the *Pediococcus acidilactici* is not particularly limited, as long as effects obtainable by the present invention are not adversely affected. The lactic acid bacterium assigned to *Pediococcus acidilactici* is preferably a *Pediococcus acidilactici* strain F-1033 deposited under accession number CECT 7483.

The *Pediococcus acidilactici* strain F-1033 is as described in Japanese Patent No. 5777640 (International Patent Publication No. WO 2011/092261). An isolation method and taxonomic characteristics thereof will be described hereinafter.

### 1. Isolation of microorganism

### A) Method

The F-1033 strain was isolated from excrement and saliva derived from children aged 0 to 5 years old (Daniel, C., et al., "Selecting Lactic Acid Bacteria for Their Safety and Functionality by Use of a Mouse Colitis Model", Appl. Environ. Microbiol., 2006, vol. 72, p. 5799-5805). The fresh excrement and saliva were dissolved in a PBS buffer (pH 7.4), pipetted, and inoculated on an MRS agar plate supplemented with a combination of various antibiotic substances. The bacterium was incubated at 37°C under microaerobic conditions (5% CO₂). Although the incubation time depends on a growth speed, it is generally 24 hours to 3 days. Gram staining was performed for initial determination. After the growth, the isolated strain was stored by freeze-drying in 0.1×PBS including 15% by mass of skim milk powder.

### B) Result

The isolated bacterium was grown on MRS agar supplemented with 10 µ g / ml vancomycin (produced by SIGMA). Microscopic examination revealed that the strain F-1033 was gram-positive in the coccoid form.

### 2. Taxonomic characteristics of strain

### 2.1. Genetic identification of genera and species

### A) Method

DNA was extracted, and the obtained DNA pellet was incubated at 65°C for 1 hour, and the resultant culture was resuspended in 100 µL of a rehydration solution to obtain a sample in the same manner as in "A) Method" of "2.1. Genetic identification of genera and species" of "2. Taxonomic characteristics of strain" of the strain I-3141, except that as the isolated bacterium, the above bacterium that was confirmed to be gram positive was used. The obtained sample was stored at 2°C to 8°C.

The 16S gene was amplified by PCR using the universal primers 27f and 1492r (Weisburg, W.G., et al., "16S ribosomal DNA amplification for phylogenetic study", J. Bacteriol., 1991, vol. 173, p. 697-703.), which generated 16S rRNA fragments (1,465 bp) of a nearly entire sequence. Next, the DNA obtained in the above-described manner was washed using QIA (registered trademark) quick kit (produced by Qiagen, GmbH, Hilden, Germany).

The primers used for amplifying and sequencing the 16S gene are presented in Table 3 below.

Four consecutive sequencing reactions were performed by Genetic Analyzer 3130 (produced by Applied Biosystems) using the primers presented in Table 3 below and a BigDye v. 3.1 kit. Data was collected and chromatograms were generated using DNA Sequence Analysis v. 5.2 software (produced by Applied Biosystems), and the results were examined by visual analysis on Chromas (produced by Technelysium Pty Ltd.) and BioEdit (produced Ibis Biosciences).

The identification of the genus and species was performed by comparing the obtained sequence with a 16S sequence of a known organism from both RefSeq database (http://www.ncbi.nlm.nih.gov/RefSeq/) of BLASTN search (Altschul, S.F., et al., "Basic local alignment search tool", J. Mol. Biol., 1990, vol. 215, p. 403-410.) and the Ribosomal Database Project tool.

**[Table 3]**

| PROCESS | PRIMER | DIRECTION | 5' →3' SEQUENCE |
|---|---|---|---|
| AMPLIFICATION | 27f | FORWARD | AGAGTTTGATCCTGGCTCAG (SEQ ID NO: 3) |
| | 1492r | REVERSE | GGTTACCTTGTTACGACTT (SEQ ID NO: 6) |
| SEQUENCING | 27f | FORWARD | AGAGTTTGATCCTGGCTCAG (SEQ ID NO: 3) |
| | 357f | FORWARD | CGCCCGCCGCGCCCCGCGCCCGGCCCGC CGCCCCCGCCCCCCTACGGGAGGCAGCAG (SEQ ID NO: 4) |
| | 907r | REVERSE | CCGTCAATTCCTTTGAGTTT (SEQ ID NO: 5) |
| | 1492r | REVERSE | GGTTACCTTGTTACGACTT (SEQ ID NO: 6) |

### B) Result

It was determined that the strain F-1033 was assigned to the *Pediococcus acidilactici* species.

### 2.2. Determination of genotype of strain

### A) Method

A genotype of the strain F-1033 was determined in the same manner as in "A) Method" of "2.2. Determination of the genotype of strain" of the strain I-3141, except that the commercially available strains of *Lacticaseibacillus casei* VSL#3 and *Lacticaseibacillus casei* DN114.001 included in the assay as control strains were changed to a commercially available strain of *Pediococcus acidilactici* Rossell 1001 (produced by Rossell (registered trademark) Institut, Canada), and the restriction enzyme used for a whole DNA digestion was changed from the restriction enzyme Sfi-I or the restriction enzyme Sma-I to a restriction enzyme Not-I or a restriction enzyme Sma-I (both produced by Roche Diagnostics).

### B) Result

As depicted in Fig. 1F, the cleavage pattern obtained by the Sma-I digestion of the strain F-1033 is similar to that of the *Pediococcus acidilactici* Rossell 1001. However, as depicted in Fig. 1E, the cleavage pattern obtained by the Not-I digestion of the strain F-1033 is clearly different from that of *Pediococcus acidilactici* Rossell 1001.

The strain F-1033 is considered as being assigned to the *Pediococcus acidilactici* from the results above. Therefore, the strain F-1033 was determined as a *Pediococcus acidilactici* strain F-1033 (may be merely referred to as a "strain F-1033" hereinafter).

Note that, the strain F-1033 was deposited at the Spanish Type Culture Collection (COLECCION ESPANOLA DE CULTIVOS TIPO (CECT)) (location: Edificio 3 CUE. Parc Cientific Universitat de Valencia Catedratico Agustin Escardino, 9 46980 Paterna(Valencia) Spain) by a depositor, AB-BIOTICS S.A. (residence: Av. Torre Blanca 57 ESADE Creapolis Building Sant Cugat del Valles 08172 Spain), and was internationally deposited on June 28, 2012 under accession number CECT 7483.

As seen in other bacteria, the strain F-1033 may have altered properties. The glucuronidase activator can include the strain F-1033 having altered properties as long as related characteristics and advantages of the glucuronidase activator described in the present specification can be retained or enhanced, and effects obtainable by the present invention are not adversely affected.

Examples of the strain F-1033 having altered properties include mutant strains derived from the strain F-1033 (e.g., natural mutant strains, and artificial mutant strains obtained by a mutation treatment with ultraviolet light, X-rays, radiation, chemicals, etc.), genetically modified products (e.g., transformants), and the like.

As a method of incubating the lactic acid bacterium assigned to the *Pediococcus acidilactici,* the lactic acid bacterium can be incubated in the same method as the method of culturing the microorganism of the present invention, and the same medium and the like can be also used.

A state of the lactic acid bacterium assigned to the *Pediococcus acidilactici* included in the glucuronidase activator is not particularly limited, and may be appropriately selected according to the intended purpose. As the state of the lactic acid bacterium assigned to the *Pediococcus acidilactici,* living bacterial cells of the lactic acid bacterium assigned to the *Pediococcus acidilactici* or a disrupted product of the bacterial cells of the lactic acid bacterium assigned to the *Pediococcus acidilactici* is preferred.

The living bacterial cells may be wet bacterial cells or dried bacterial cells. Specific examples of the living bacterial cells include a culture of the lactic acid bacterium assigned to the *Pediococcus acidilactici,* a concentrate of the culture, a dried product of the culture, a diluted product of the culture, and the like.

In view of enhancement of glucuronidase activity, the glucuronidase activator preferably includes three kinds of lactic acid bacteria, which are the strain I-3141, the lactic acid bacterium assigned to the *Lactoplantibacillus plantarum,* and the lactic acid bacterium assigned to the *Pediococcus acidilactici,* and more preferably includes three kinds of lactic acid bacteria, which are the strain I-3141, *Lactoplantibacillus plantarum* deposited under accession number CECT 7481, and *Pediococcus acidilactici* deposited under accession number CECT 7483.

An amount of the lactic acid bacterium assigned to the *Lactoplantibacillus plantarum* and the lactic acid bacterium assigned to the *Pediococcus acidilactici* in the glucuronidase activator is not particularly limited as long as effects obtainable by the present invention are not adversely affected, and may be appropriately selected according to the intended purpose.

### -Use-

Since the glucuronidase activator has excellent glucuronidase activity, the glucuronidase activator can be used, for example, for increasing an estrogen level in a body, improving estrogen metabolism in a body, improving climacteric symptoms, or enhancing glucuronidase activity in an intestinal tract. Therefore, a method of increasing an estrogen level in a body through administration of the glucuronidase activator, a method of improving estrogen metabolism through administration of the glucuronidase activator, the method of improving climacteric symptoms through administration of the glucuronidase activator, and a method of enhancing glucuronidase activity through administration of the glucuronidase activator are within the scope of the present invention.

Moreover, the glucuronidase activator is suitably used for the below-described pharmaceutical composition, probiotic composition, edible composition, and the like, and can be used as a reagent for research associated with the glucuronidase activity.

Usage, a dosage form, a dose to an individual, administration interval, and an individual to be a subject are not particularly limited, and may be appropriately selected according to an age, condition, and body weight of an administration subject, absorption of the active ingredient within a body, inactivity of the active ingredient, an excretion rate of the active ingredient, the presence or absence of a drug used in combination, a kind of the drug used in combination, and the like.

The usage of the glucuronidase activator is not particularly limited, and may be appropriately selected according to the intended purpose. Examples of the usage thereof include oral administration, parenteral administration, and the like.

Examples of the parenteral administration include percutaneous administration, enteral administration, transmucosal administration, intravenous administration, intraarterial administration, subcutaneous administration, intramuscular administration, and the like.

Among the above, oral administration is preferred.

The dosage form of the glucuronidase activator is not particularly limited, and may be appropriately selected from dosage forms available in the related art according to the intended usage. The dosage form may be a solid form, a semisolid form, or a liquid form. Examples thereof include: oral preparations, such as tablets, powders, capsules, soft capsules, granules, powders, oral liquids, elixirs, syrups, drinks, troches, mouthwashes, and the like; and parenteral preparations such as injections, drips, sprays, inhalation sprays, suppositories, and the like.

The glucuronidase activator in any of the above dosage forms can be produced by a method known in the related art.

As an individual as a subject of the glucuronidase activator, the glucuronidase activator is suitably applied to humans, but can be also applied to animals other than humans (e.g., mice, rats, hamsters, dogs, cats, cows, pigs, monkeys, horses, goats, birds, etc.) as long as effects are inhibited therein.

### (Pharmaceutical composition)

The pharmaceutical composition of the present invention includes a glucuronidase activator and a pharmaceutically acceptable carrier, and may further include other ingredients as necessary.

Note that, the pharmaceutical composition is not limited to any category, such as a drug, quasi-drug, or the like, according to the administrative category, and may be either a drug or a quasi-drug.

### <Glucuronidase activator>

The glucuronidase activator included in the pharmaceutical composition is as described in the above section of (Glucuronidase activator).

An amount of the glucuronidase activator in the pharmaceutical composition is not particularly limited, and may be appropriately selected according to the intended purpose.

### <Pharmaceutically acceptable carrier>

The pharmaceutically acceptable carrier is not particularly limited, and may be appropriately selected according to the intended purpose. Examples of the pharmaceutically acceptable carrier include additives, auxiliary agents, solvents, and the like. The above pharmaceutically acceptable carriers may be used alone or in combination.

The additives or the auxiliary agents are not particularly limited, and may be appropriately selected according to the intended purpose. Examples of the additives or the auxiliary agents include excipients, fungicides, preservatives, binding agents, thickeners, fixing agents, binders, colorants, stabilizers, pH adjusters, buffers, isotonic agents, solvents, antioxidants, ultraviolet inhibitors, crystallization inhibitors, antifoaming agents, physical property improvers, antiseptics, and the like.

The excipient is not particularly limited, and may be appropriately selected according to the intended purpose. Examples of the excipient include D-mannitol, sucrose (including purified sucrose), sodium hydrogen carbonate, starch **(e.g.,** corn starch, potato starch, wheat starch, rice starch, partially pregelatinized starch, etc.), crystalline cellulose, light anhydrous silicic acid, calcium (anhydrous calcium hydrogen phosphate, precipitated calcium carbonate, calcium silicate, and calcium stearate, etc.), and the like.

The solvent is not particularly limited, and may be appropriately selected according to the intended purpose. Examples of the solvent include water, physiological saline, buffer solutions, and the like.

An amount of the pharmaceutically acceptable carrier in the pharmaceutical composition is not particularly limited as long as effects obtainable by the present invention are not adversely affected, and may be appropriately selected according to the intended purpose.

The pharmaceutical composition may be used alone, or in combination with a drug including another substance as an active ingredient. Moreover, the pharmaceutical composition may be used in a state where the pharmaceutical composition is formulated in a drug including another substance as an active ingredient, or the like.

Usage, a dosage form, a dose to an individual, administration interval, and an individual to be a subject are not particularly limited, and may be appropriately selected according to the intended purpose. For example, the same usage, dosage form, dose to an individual, administration interval and individual as those of the glucuronidase activator may be used. Among the above, the usage of the pharmaceutical composition is preferably oral administration, and the dosage form of the pharmaceutical composition is preferably an oral preparation. Accordingly, the pharmaceutical composition is suitably used as an oral composition.

Moreover, the pharmaceutical composition in any of the above dosage forms can be produced by a method known in the related art.

### -Use-

Since the pharmaceutical composition includes the glucuronidase activator having excellent glucuronidase activity, the pharmaceutical composition can be suitably used, for example, for increasing an estrogen level in a body, improving estrogen metabolism in a body, improving climacteric symptoms, or enhancing glucuronidase activity in an intestinal tract.

### (Probiotic composition)

The probiotic composition of the present invention includes a glucuronidase activator, and may further include other ingredients as necessary.

In the present specification, the term "probiotics" means bacteria that demonstrate beneficial functions in a gut.

### <Glucuronidase activator>

The glucuronidase activator included in the probiotic composition is as described in the above section of (Glucuronidase activator).

An amount of the glucuronidase activator in the probiotic composition is not particularly limited, and may be appropriately selected according to the intended purpose. Moreover, the probiotic composition may be the glucuronidase activator itself.

### <Other ingredients>

As other ingredients included in the probiotic composition, the same ingredients described in the above section of <Other ingredients> of (Glucuronidase activator).

An amount of the above other ingredients in the probiotic composition is not particularly limited as long as effects obtainable by the present invention are not adversely affected, and may be appropriately selected according to the intended purpose.

Usage, a dosage form, a dose to an individual, administration interval, and an individual to be a subject are not particularly limited, and may be appropriately selected according to the intended purpose. For example, the same usage, dosage form, dose to an individual, administration interval, and individual to be a subject as those of the glucuronidase activator may be used. Among the above, the usage of the probiotic composition is preferably oral administration, and the dosage form of the probiotic composition is preferably an oral preparation. Accordingly, the probiotic composition can be suitably used as an oral composition.

Moreover, the probiotic composition in any of the above dosage forms can be produced by a method known in the related art.

### -Use-

Since the probiotic composition includes the glucuronidase activator having excellent glucuronidase activity, the probiotic composition can be suitably used, for example, for increasing an estrogen level in a body, improving estrogen metabolism in a body, improving climacteric symptoms, or enhancing glucuronidase activity in an intestinal tract.

### (Edible composition)

The edible composition of the present invention includes the glucuronidase activator and an edible ingredient, and may further include other ingredients as necessary.

The edible composition is a composition that hardly harms human health and is orally ingested in ordinary social life. Therefore, the edible composition means a wide range of food products constituting orally ingested general food products, health foods (functional foods and drinks), health functional foods (foods for specific health benefits, nutrient functional foods, and foods with functional claims), foods for patients, and the like. Moreover, the edible composition also includes beverages.

### <Glucuronidase activator>

The glucuronidase activator included in the edible composition is as described in the above section of (Glucuronidase activator).

An amount of the glucuronidase activator in the edible composition is not particularly limited, and may be appropriately selected according to the intended purpose.

### <Edible ingredients>

In the present specification, the "edible ingredient" means an ingredient suitable for edible purposes, which contains nutrients, such as proteins, lipids, carbohydrates, calcium, vitamins, carotines, and the like, other than substances included in the glucuronidase activator. The ingredient suitable for edible purposes is preferably an ingredient suitable for consumption by humans or animals other than humans.

An amount of the edible ingredient in the edible composition is not particularly limited, and may be appropriately selected according to an intended kind of the edible composition.

The kind of the edible composition is not particularly limited, and may be appropriately selected according to the intended purpose. Examples of the kind of the edible composition include: beverages such as lactic acid bacteria beverages, tea beverages, soft drinks, carbonated beverages, nutritional beverages, fruit beverages, and the like; frozen desserts, such as ice cream, sherbet, and shaved ice; noodles, such as buckwheat noodles, udon noodles, starch noodle, Gyoza skins, Shumai skins, Chinese noodles, instant noodles, and the like; confectionary, such as hard candies, candies, chewing gums, chocolate, tablets, snacks, biscuits, jelly, jams, creams, sweet pastries, bread, and the like; fisheries or processed products thereof, such as crabs, salmon, clams, tuna, sardine, shrimps, bonito, mackerel, whale, oysters, Pacific saury, squid, Anadora broughtonii, scallops, abalone, sea urchin, salmon roe, Sulculus diversicolor supertexta, and the like; fish or meat processed foods, such as fish paste, ham, sausages, and the like; dairy products, such as fermented milk, fermented soybean milk, processed milk, fermented milk, yogurt, and the like; fat or oil, or fat or oil processed food products, such as salad oil, tempura oil, margarines, mayonnaise, shortening, whipping creams, dressing, and the like; seasonings, such as sources and dip sources; retort pouch-packaged foods, such as curry, stew, Oyakodon, gruel, rice soups, Chukadon, a bowl of rice with breaded pork cutlets, a bowl of rice with tempura, Unadon, Hayashi rice, Oden, Mapo tofu, a bowl of rice with beef, Bolognese sauces, egg soup, omelet rice, Gyoza, Shumai, hamburgers, meat balls, and the like; daily dishes, such as salads and pickles; various forms of health, cosmetic, or nutritional supplementary foods; and the like.

A method of producing the edible composition is not particularly limited as long as the glucuronidase activator can be included, and may be appropriately selected from methods known in the related art according to an intended kind of the edible composition.

The edible composition may be packaged in a container. Moreover, the container may include a label or instruction, which indicates, as a function of the edible composition, at least one selected from the group consisting of:
(a) increase of an estrogen level in a body;
(b) improvement of estrogen metabolism; and
(c) improvement of climacteric symptoms.

### -Use-

Since the edible composition includes the glucuronidase activator having excellent glucuronidase activity, the edible composition can be used, for example, for increasing an estrogen level in a body, improving estrogen metabolism in a body, improving climacteric symptoms, or enhancing glucuronidase activity in an intestinal tract. Moreover, the edible composition can be also suitably used as a probiotic composition.

### (Oral composition)

The oral composition of the present invention includes a *Levilactobacillus brevis* strain I-3141 deposited under accession number CECT 7480, *Lactoplantibacillus plantarum* deposited under accession number CECT 7481, and *Pediococcus acidilactici* deposited under accession number CECT 7483, and may further include other ingredients as necessary.

The *Levilactobacillus brevis* strain I-3141 deposited under accession number CECT 7480, the *Lactoplantibacillus plantarum* deposited under accession number CECT 7481, and the *Pediococcus acidilactici* deposited under accession number CECT 7483 in the oral composition are as described in the above section of (Glucuronidase activator).

### <Other ingredients>

As other ingredients included in the oral composition, the same ingredients described in the above section of <Other ingredients> of (Glucuronidase activator), or the same ingredients described in the above section of <Edible ingredients> of (Edible composition).

An amount of the above other ingredients in the oral composition is not particularly limited as long as effects obtainable by the present invention are not adversely affected, and may be appropriately selected according to the intended purpose.

### (Microorganism-containing composition)

The microorganism-containing composition of the present invention includes a *Levilactobacillus brevis* strain I-3141 deposited under accession number CECT 7480, and may further include other ingredients as necessary.

### <Strain I-3141>

The strain I-3141 included in the microorganism-containing composition is as described in the above section of (Microorganism).

An amount of the strain I-3141 in the microorganism-containing composition is not particularly limited, and may be appropriately selected according to the intended purpose. Note that, the microorganism-containing composition may be the strain I-3141 itself.

### <Other ingredients>

The above other ingredients in the microorganism-containing composition are not particularly limited as long as effects obtainable by the present invention are not adversely affected, and may be appropriately selected according to the intended purpose. For example, the same ingredients described in the above section of <Other ingredients> of (Glucuronidase activator) can be used.

Moreover, the microorganism-containing composition may include other microorganisms.

The other microorganisms preferably include three kinds of lactic acid bacteria, which include the lactic acid bacterium assigned to the *Lactoplantibacillus plantarum* and the lactic acid bacterium assigned to the *Pediococcus acidilactici,* and more preferably includes three kinds of lactic acid bacteria, which include *Lactoplantibacillus plantarum* deposited under accession number CECT 7481, and *Pediococcus acidilactici* deposited under accession number CECT 7483.

A method of producing the microorganism-containing composition is not particularly limited, as long as the strain I-3141 can be included, and the method may be appropriately selected from methods available in the related art according to the intended purpose.

### -Use-

The microorganism-containing composition can be suitably used for the probiotic composition, the glucuronidase activator, the pharmaceutical composition, the edible composition, and the like.

### Examples

The present invention will be concretely described hereinafter through Test Examples and Production Examples, but the present invention is not limited to Test Examples and Production Examples.

### (Test Example 1)

Lactic acid bacteria having β-glucuronidase activity, which could deconjugate a glucuronic acid conjugate of estrogen, were selected by the following method.

Using an MRS liquid medium (BD Difco^{™} Lactobacillus MRS broth, produced by Becton Dickinson), 375 strains of lactic acid bacteria including the strain NBRC 107147 (available from NBRC), which was a type strain of *Levilactobacillus brevis,* were each precultured (static culture) at 37°C for 24 hours.

Next, each of the lactic acid bacteria precultured in the MRS liquid medium was added at a final concentration of 0.1% (v/v) into an MRS liquid culture (BD Difco^{™} Lactobacillus MRS broth, produced by Becton Dickinson) to which 5-bromo-4-chloro-3-indolyl-β-D-glucuronide cyclohexylammonium (X-Gluc) (produced by FUJIFILM Wako Pure Chemical Corporation) was added at a final concentration of 200 µg/mL, and statically cultured at 37°C for 72 hours.

After completing the culture, the color of the culture solution was observed. The lactic acid bacterium whose culture solution had the color of blue to blue-green due to the presence of a blue dye (5,5'-dibromo-4,4'-dichloro-indigo) generated by hydrolysis of X-Gluc was selected as a lactic acid bacterium having GUS activity.

The selected lactic acid bacterium was determined as the strain I-3141 (accession number: CECT 7480). The mycological properties of the strain I-3141 are as described in the above section of (Microorganism), and is a lactic acid bacterium assigned to *Levilactobacillus brevis.*

Moreover, it was found that the strain NBRC 107147 that was a type strain of *Levilactobacillus brevis* also had GUS activity.

### (Test Example 2)

The strain I-3141 selected in Test Example 1 and the strain NBRC 107147 as a type strain were used to confirm the GUS activity and specificity by the following method.

### <Preparation of bacterial cell lysate>

Using an MRS liquid medium (BD Difco^{™} Lactobacillus MRS broth, produced by Becton Dickinson), each of the strain I-3141 and NBRC 107147 was precultured (static culture) at 37°C for 24 hours.

Next, each of the lactic acid bacteria precultured in MRS liquid medium was added at a final concentration of 0.1% (v/v) into an MRS liquid (BD Difco^{™} Lactobacillus MRS broth, produced by Becton Dickinson), and cultured at 37°C for 48 hours.

After completing the culture, the culture solution was centrifuged, and the culture supernatant was removed to obtain cells of the cultured lactic acid bacterium. To the cells of the lactic acid bacterium, GUS buffer (100 mM sodium phosphate buffer solution added with 2.5 mM ethylene diamine tetraacetate, pH 6.0) was added in an amount equal to the removed culture supernatant. After washing the cells, centrifugation was performed, and the supernatant was removed. The second washing was performed in the same manner as above, to thereby obtain the cells of the lactic acid bacterium.

The GUS buffer was added to the obtain cells of the lactic acid bacterium in an amount 24 times (v/w) the amount of the cells to suspend the cells, followed by adding glass beads in an amount 20 times (w/w) the amount of the cells, and then the cells were disrupted by Multi-beads shocker (registered trademark) (produced by Yasui Kikai Corporation). Next, the disrupted cell suspension was centrifuged to obtain a supernatant (bacterial cell lysate of the lactic acid bacterium).

### <Evaluation of GUS activity>

To the obtained the bacterial cell lysate (adjusted to a final protein concentration of 0.25 mg/mL with the GUS buffer) of each lactic acid bacteria, 4-nitrophenyl β-D-glucuronide (pNGP) (produced by FUJIFILM Wako Pure Chemical Corporation), estrone 3-(β-D-glucuronide) (E1-3G) (FUJIFILM Wako Pure Chemical Corporation), or estradiol 3-(β-D-glucuronide) (E2-3G) (produced by FUJIFILM Wako Pure Chemical Corporation) serving as a substrate was added at a final concentration of 1.0 mM, followed by reacting at 37°C. The concentration of the product generated due to the GUS activity was measured at the start of the reaction (0 minutes), 15 minutes, 30 minutes, 45 minutes, and 60 minutes after the start of the reaction, to evaluate the GUS activity. Moreover, as a negative control, the same test was performed using only the GUS buffer instead of the bacterial cell lysate, and the substrate.

Specifically, in the case where 4-nitrophenyl β-D-glucuronide (pNGP) was used as the substrate, absorbance at 405 nm was measured by a microplate reader every 15 minutes after the start of the reaction. A concentration of pNP generated by the GUS activity was calculated based on the value of the absorbance of 4-nitrophenol (pNP) (produced by FUJIFILM Wako Pure Chemical Corporation) of various concentrations at 405 nm that were separately measured.

Moreover, in the case where estrone 3-(β-D-glucuronide) (E1-3G) or estradiol 3-(β-D-glucuronide) (E2-3G) was used as the substrate, a 25% by mass trichloroacetate solution (produced by FUJIFILM Wako Pure Chemical Corporation) was added in an amount equal to the amount of the reaction solution every 15 minutes after the start of the reaction, to stop the reaction. After the reaction was stopped, a supernatant was recovered by centrifugation, and the concentration of E1-3G or E2-3G in the supernatant was determined by high-performance liquid chromatography (HPLC) under the following conditions. The concentration of estrone (E1) or estradiol (E2) generated by the GUS activity was calculated from the reduction in the concentration of E1-3G or E2-3G.

The results are presented in Table 4 below and Figs. 2 to 4.
[HPLC analysis conditions]
Device: Prominence (manufactured by Shimadzu Corporation) Column: YMC-Pack ODS-A (particle diameter of 5 µm, pore diameter of 12 nm, 4.6 mm in inner diameter × 250 mm in length) (YMC CO., LTD.)
Column temperature: 40°C
Mobile phase: 0.1% by volume formic acid-acetonitrile solution : 0.1% by volume formic acid aqueous solution = 45:55 (v/v) Injection amount: 10 µL
Flow rate: 0.7 mL/min
Detector: photodiode array detector SPD-M20A (manufactured by Shimadzu Corporation)

**[Table 4]**

| STRAIN | SUBSTRATE | PRODUCT | CONCENTRATION OF PRODUCT (mM) | | | | |
|---|---|---|---|---|---|---|---|
| | | | AT START OF REACTION (0 mins. ) | 15 MINUTES AFTER START OF REACTION | 30 MINUTES AFTER START OF REACTION | 45 MINUTES AFTER START OF REACTION | 60 MINUTES AFTER START OF REACTION |
| *Levilactobacillus brevis* | pNPG | pNP | 0.00 | 0.28 | 0.46 | 0.59 | 0.69 |
| CECT 7480 | E1-3G | E1 | 0.00 | 0.28 | 0.56 | 0.67 | 0.74 |
| STRAIN I-3141 | E2-3G | E2 | 0.00 | 0.32 | 0.50 | 0.69 | 0.75 |
| *Levilactobacillus brevis* | pNPG | pNP | 0.00 | 0.17 | 0.32 | 0.44 | 0.53 |
| STRAIN NBRC 107147 | E1-3G | E1 | 0.00 | 0.15 | 0.25 | 0.34 | 0.44 |
| | E2-3G | E2 | 0.00 | 0.15 | 0.28 | 0.37 | 0.45 |
| NEGATIVE CONTROL | pNPG | pNP | 0.00 | 0.00 | 0.00 | 0.00 | 0.00 |
| | E1-3G | E1 | 0.00 | 0.00 | 0.00 | 0.00 | 0.00 |
| | E2-3G | E2 | 0.00 | 0.00 | 0.00 | 0.00 | 0.00 |

It was found from the results of Table 4 and Figs. 2 to 4 that the strain I-3141 had a high concentration of the product with all of the three substrates, and had excellent glucuronidase activity compared to the strain NBRC 107147 that was the type strain.

### (Test Example 3)

An effect on estrogen in blood was tested using the strain I-3141 selected in Test Example 1 by the following method.

Nine week-old female germ-free mice (strain name: C57BL/6N Jcl) (CLEA Japan, Inc.) were divided into two groups, i.e., a solvent control group and an I-3141 administration group,

The mice (n = 7) of the solvent control group were orally administered with physiological saline three times a week over a period of two weeks.

The mice (n = 7) of the I-3141 administration group were orally administered with the strain three times a week over a period of two weeks. For the administration, the strain I-3141 was suspended in physiological saline, and the suspension was administered at 1×10⁹ CFU per mice per administration.

Two weeks after the administration (when the mice of each group were 11 weeks old), blood was collected and serum was separated from the collected blood. A concentration of estradiol (E2) in the serum was measured by liquid chromatography-tandem mass spectrometry (LC-MS/MS) (the LC-MS/MS analysis was outsourced to Asuka Pharmaceutical Medical Co., Ltd.) according to the method described in Japanese Patent No. 6041125. A specific measuring method will be described below.

In Test Example 3, the measurement of the concentration of the estradiol (E2) in the serum was performed only on the individual who was determined to be in the estrus phase by the following method.

At the same time as the collection of the blood, a vaginal smear was collected. The vaginal smear was applied to a slide glass, dried, and then stained with Giemsa to prepare a vaginal smear image. A concentration of estradiol was measured on the individuals (solvent control group: 3 mice, I-3141 administration group: 5 mice) who were determined as in the estrus phase from their vaginal smear images by the following method, and an average value of each group was calculated. A difference between the two groups was analyzed using Student's t-test, and a p value (probability value) of p < 0.05 was considered as statistically significant. The results are presented in Table 6 below.

### <Preparation of calibration curve samples>

As calibration curve samples, estradiol E2 (produced by FUJIFILM Wako Pure Chemical Corporation) methanol solutions (0.01 pg/50 µL, 0.05 pg/50 µL, 0.1 pg/50 µL, 1 pg/50 µL, 10 pg/50 µL, and 100 pg/50 µL) were respectively added in an amount of 50 µL to separate test tubes, and pure water was added to increase the total volume to be 1 mL.

### <Preparation of serum samples for measurement>

### <<Step 1: elution of estradiol (E2)>>

To 0.075 mL of the mouse serum diluted with 1 mL of pure water, 10 pg/50 µL of an internal standard (E2-¹³C₄) was added, followed by adding 4 mL of methyl tert-butyl ether. The resultant mixture was mixed by shaking for 5 minutes. Then, the aqueous phase was separated by freezing to remove the solvent of the oil phase. Thereafter, the residue was dissolved in 0.5 mL of methanol, and 1 mL of pure water was added to the solution. The resultant solution was added to Oasis (registered trademark) MAX cartridge (produced by Waters), which had been conditioned in advance with 3 mL of methanol, followed by conditioning with 3 mL of pure water. Thereafter, the cartridge was washed with 1 mL of 1% by volume acetic acid, 1 mL of 30% by volume acetonitrile, 1 mL of 1 M sodium hydroxide, 3 mL of methanol, 1 mL of 1% by volume acetic acid, and 1 mL of pyridine/60% by volume methanol (1:100 (volume ratio)), and then estradiol (E2) was eluted with 1 mL of pyridine/90% by volume methanol (1:100 (volume ratio)). Then, the eluate was removed by a centrifugal evaporator.

### <<Step 2: preparation of E2-3-tetrafluoropyridine ether>>

To the residue obtained in Step 1, 0.05 mL of pentafluoropyridine, 0.1 mL of acetonitrile, and 0.02 mL of triethylamine were added, and the resultant mixture was sufficiently mixed by shaking, followed by leaving to stand for 20 minutes at room temperature. After the reaction, the reaction reagents were removed under reduced pressure.

### <<Step 3: Elution of E2-3-tetrafluoropyridyl ether>>

To the residue obtained in Step 2, 0.14 mL of a solution in which 1 mg of 2-hydrazino-1-methylpyridine was dissolved in 1% by volume trifluoroacetate-acetonitrile (1:1,400 (volume ratio)) was added, and the resultant mixture was left to stand for 1 hour at room temperature. Note that, the production method of 2-hydrazino-1-methylpyridine is described in Japanese Unexamined Patent Application Publication No. 2010-36810.

After the reaction, the solvent was removed under reduced pressure. After dissolving the residue in 0.5 mL of methanol, 0.5 mL of pure water was added. The resultant solution was added to Oasis (registered trademark) WCX cartridge, which had been conditioned in advance with 3 mL of methanol, followed by conditioned with 3 mL of pure water. Thereafter, the cartridge was washed with 1 mL of 0.1 M potassium dihydrogen phosphate, 2 mL of pure water, and 1 mL of 40% by volume acetonitrile, and then E2-3-tetrafluoropyridyl ether was eluted with 1 mL of methanol. Then, the solvent was removed under reduced pressure.

### <<Step 4: production of E2-3-tetrafluoropyridyl ether-17-fusaric acid derivative>>

After drying the residue including E2-3-tetrafluoropyridyl ether obtained in Step 3 for 30 minutes or longer under reduced pressure, 0.05 mL of derivatization reagents (40 mg of fusaric acid, 40 mg of 2-methyl-6-nitrobenzoic anhydride, 20 mg of 4-dimethylaminopyridine/1 mL of acetonitrile) and 0.01 mL of triethylamine were added, and the resultant mixture was left to stand for 30 minutes at room temperature. After the reaction, 0.5 mL of hexane/acetic acid (50:1 (volume ratio)) was added to the reaction solution, and the resultant mixture was sufficiently mixed by shaking. The resultant solution was added to InertSep (registered trademark) SI cartridge, which had been conditioned in advance with 3 mL of ethyl acetate, followed by conditioning with 3 mL of hexane. Thereafter, the cartridge was washed with 1 mL of hexane and 2 mL of ethyl acetate/hexane (15:85 (volume ratio)), and then an E2-3-tetrafluoropyridyl ether-17-fusaric acid derivative (E2-TfpyFU) was eluted with ethyl acetate/hexane (45:55 (volume ratio)). Then, the eluate was removed under reduced pressure. The residue was dissolved in 0.1 mL of 80% by volume acetonitrile to prepare an E2-TfpyFU solution.

### <Measurement of estradiol (E2) concentration>

The E2-TfpyFU solution (0.02 mL) obtained in Step 4 was injected to LC-MS/MS, and a measurement was performed under the measurement conditions presented in Table 5 below. Intact protonated ions were detected at m/z583 on the ESI mass spectrum of E2-TfpyFU measured by the mass spectrometer. Further, MS/MS was performed using m/z583 as a precursor ion. As a result, the m/z308 was the product ion having the highest specificity, and therefore, these ions were used for selective reaction monitoring (SRM) to perform quantification.

**[Table 5]**

| MEASUREMENT CONDITIONS OF LC-MS/MS OF E2-TfpyFU | | | |
|---|---|---|---|
| HPLC CONDITIONS | | | |
| | AUTO SAMPLER | | **: SHIMADZU SIL HTC** |
| | PUMP | | **: SHIMADZU LC-20AD** |
| | 1st COLUMN | | **: Acentis Express Phenyl-Hexyl (2. 7 *µ*m, 2. 0×100 mm)** |
| | 2nd COLLUMN | | **Kinetex C18 (2. 6 *µ*m, 2. 0×100 mm)** |
| | MOBILE PHASE (1st) | | **: Acetonitrile-H₂O** SOLUTION (3:1) |
| | MOBILE PHASE (2nd) | | **: A: H₂O** |
| | | | **B: Acetonitrile** |
| | | | **0. 00→2. 00(min)A/B=0:100** |
| | | | **2. 00→2. 01(min)A/B=0:100-30:70** |
| | | | **2. 01→5. 80(min)A/B=30:70** |
| | | | **5. 80→6. 00(min)A/B= 30:70-10:90** |
| | | | **6. 00→7. 50(min)A/B=10:90→0:100** |
| | | | **7. 50→7. 70(min)A/B=0:100** |
| | COLUMN TEMPERATURE | | : **40°C** |
| | FLOW RATE (1st) | | **: 0. 25 mL/min** |
| | FLOW RATE (2nd) | | **: 0. 5 mL/min** |
| | INJECTION AMOUNT | | **: 0. 02mL** (DISSOLVED IN 0.1 mL of 80% ACETONITRILE) |
| | **Run Time** | | **: 7. 7 min** |

| MS/MS CONDITIONS | | | |
|---|---|---|---|
| | **MS/MS** | | **: API5000(AB Sciex)** |
| | IONIZATION | | **: ESI (Positive)** |
| | | **CAD** | **: 6 psi** |
| | | **CUR** | **: 12 psi** |
| | | **Gas 1** | **: 20 psi** |
| | | **Gas 2** | **: 70 psi** |
| | | **IonSpray Voltage** | **: 5500 volts** |
| | | ION SOURCE TEMPERATURE | **: 600 °C** |

| MEASURED ION(***m*/*z***) | | | |
|---|---|---|---|
| | | **E2-TfpyFU** | **: 583 → 308** |
| | | **E2-¹³C₄-TfpyFU** | **: 587 → 311** |

**[Table 6]**

| GROUP | ESTRADIOL (E2) CONCENTRATION (pg/mL) |
|---|---|
| SOLVENT CONTROL GROUP | 2.0±0.6 |
| STRAIN I-3141 ADMINISTRATION GROUP | 5.3±2.1* |

| | |
|---|---|
| * : p<0.05 | |

It was found from the results of Table 6 that administration of the strain I-3141 significantly increased the concentration of estradiol in blood.

### (Production Example 1)

A food product including the strain I-3141 selected in Test Example 1 was produced by the following method.

Based on the formulation below, a mixed powder of the following three kinds of lactic acid bacteria (available from AB-BIOTICS S.A.), and dry starch and calcium stearate serving as excipients were mixed to thereby obtain a powder composition for filling capsules. Subsequently, hard capsules (QUALI-V (registered trademark)-N, produced by Qualicaps Co., Ltd.) were filled with 280 mg of the powder composition per capsule using a capsule filling machine (LIQFIL-superJCF, produced by Qualicaps Co., Ltd.) to thereby produce a food product.

### <Formulation of powder composition>

Mixed powder of three kinds of lactic acid bacteria (dry powder of the strain I-3141 (0.5×10⁹ CFU/capsule), *Lactoplantibacillus plantarum* CECT 7481 (0.25×10⁹ CFU/capsule), and *Pediococcus acidilactici* CECT 7483 (0.25×10⁹ CFU/capsule)): 75 mg
Dry starch (dry potato starch, produced by Matsutani Chemical Industries, Ltd.): 199 mg
Calcium stearate (calcium stearate (plant-derived), produced by Taihei Chemical Industry Co., Ltd.): 6 mg

### (Test Example 4)

An effect of the food product on estrogen in blood was tested using the food product produced in Production Example 1 (may be referred to as "test food product" hereinafter) by the following method.

### <Production of control food product>

A control food product was produced in the same manner as in Production Example 1, except that the three kinds of lactic acid bacteria were not formulated, and 75 mg of the total amount of the formulated amount of the three kinds of lactic acid bacteria was changed to 75 mg of dry starch (dry potato starch, produced by Matsutani Chemical Industries, Ltd.).

### <Test method>

One hundred and eleven peri- menopausal women were divided into two group, i.e., a control food product intake group (57 women, average age of 49.95 ± 0.32 years old) and a test food product intake group (54 women, average age of 50.35 ± 0.40 years old). The control food product intake group was allowed to take one capsule, once a day, after breakfast through oral administration for 12 consecutive weeks. The test food product intake group was allowed to take one capsule of the test food product, once a day, through oral administration for 12 consecutive weeks. Blood was collected at the start of the test (before the first intake) and after the end of the 12 weeks of intake, and serum was separated from the collected blood.

### <<Measurement of estradiol (E2) concentration>>

The concentration of estradiol (E2) in the serum was measured in the same manner as in Test Example 3.

### <<Measurement of estrone (E1) concentration>>

A concentration of estrone (E1) in the serum was measured by liquid chromatography-tandem mass spectrometry (LC-MS/MS) (the LC-MS/MS analysis was outsourced to Asuka Pharmaceutical Medical Co., Ltd.) according to the method described in Japanese Patent No. 6041125. A specific measuring method will be described below.

### <Preparation of calibration curve samples>

As calibration curve samples, estrone (E1) (produced by FUJIFILM Wako Pure Chemical Corporation) methanol solutions (0.05 pg/50 µL, 0.1 pg/50 µL, 0.25 pg/50 µL, 1, 10 pg/50 µL, and 100 pg/50 µL), were respectively added in an amount of 50 µL to separate test tubes, and pure water was added to increase the total volume to be 1 mL.

### <Preparation of serum samples for measurement>

### <<Step 1: elution of estrone (E1)>>

To 0.075 mL of the mouse serum diluted with 1 mL of pure water, 10 pg/50 µL of an internal standard (E1-¹³C₄) was added, followed by adding 4 mL of methyl tert-butyl ether. The resultant mixture was mixed by shaking for 5 minutes. Then, the aqueous phase was separated by freezing to remove the solvent of the oil phase. Thereafter, the residue was dissolved in 0.5 mL of methanol, and 1 mL of pure water was added to the solution. The resultant solution was added to Oasis (registered trademark) MAX cartridge (produced by Waters), which had been conditioned in advance with 3 mL of methanol, followed by conditioning with 3 mL of pure water. Thereafter, the cartridge was washed with 1 mL of 1% by volume acetic acid, 1 mL of 30% by volume acetonitrile, 1 mL of 1 M sodium hydroxide, 3 mL of methanol, 1 mL of 1% by volume acetic acid, and 1 mL of pyridine/60% by volume methanol (1:100 (volume ratio)), and then estrone (E1) was eluted with 1 mL of pyridine/90% by volume methanol (1:100 (volume ratio)). Then, the eluate was removed by a centrifugal evaporator.

As described in Test Example 3, according to the above method, estradiol (E2) was eluted simultaneously with the elution of the estrone (E1).

### <<Step 2: production of E1-3-tetrafluoropyridyl ether>>

To the residue obtained in Step 1, 0.05 mL of pentafluoropyridine, 0.1 mL of acetonitrile, and 0.02 mL of triethylamine were added, and the resultant mixture was sufficiently mixed by shaking, followed by leaving to stand for 20 minutes at room temperature. After the reaction, the reaction reagents were removed under reduced pressure.

### <<Step 3: elution of E2-3-tetrafluoropyridyl ether>>

To the residue obtained in Step 2, 0.14 mL of a solution in which 1 mg of 2-hydrazino-1-methylpyridine was dissolved in 1% by volume trifluoroacetate-acetonitrile (1:1,400 (volume ratio)) was added, and the resultant mixture was left to stand for 1 hour at room temperature. After the reaction, the solvent was removed under reduced pressure. After dissolving the residue in 0.5 mL of methanol, 0.5 mL of pure water was added. The resultant solution was added to Oasis (registered trademark) WCX cartridge, which had been conditioned in advance with 3 mL of methanol, followed by conditioned with 3 mL of pure water. Thereafter, the cartridge was washed with 1 mL of 0.1 M potassium dihydrogen phosphate, 2 mL of pure water, and 1 mL of 40% by volume acetonitrile, and then E2-3-tetrafluoropyridyl ether was eluted with 1 mL of methanol. Then, the solvent was removed under reduced pressure.

Subsequently, the cartridge was washed with 2 mL of acetonitrile, 0.5 mL of pure water, and 1 mL of formic acid/65% by volume methanol (1:50 (volume ratio)), an E1-3-tetrafluoropyridyl ether-17-(1'-methylpyridinium-2')-hydrazone derivative (E1-TfpyHMP) was eluted with 1 mL of formic acid/methanol (1:50 (volume ratio)). The eluate was removed by removing the solvent under reduced pressure. The residue was dissolved in 0.1 mL of 70% by volume acetonitrile to prepare an E1-TfpyHMP solution.

### <Measurement of estrone (E1) concentration>

The E1-TfpyHMP solution (0.02 mL) obtained in Step 3 was injected to LC-MS/MS, and a measurement was performed under the measurement conditions presented in Table 7 below. Intact protonated ions were detected at m/z525 on the ESI mass spectrum of E1-TfpyHMP measured by the mass spectrometer. Further, Ms/MS was performed using m/z525 as a precursor ion. As a result, the m/z308 was the product ion having the highest specificity, and therefore, these ions were used for selective reaction monitoring (SRM) to perform quantification.

**[Table 7]**

| MEASUREMENT CONDITIONS OF LC-MS/MS OF E1-TfpyHMP | | | |
|---|---|---|---|
| HPLC CONDITIONS | | | |
| | AUTO SAMPLER | | **: SHIMADZU SIL HTC** |
| | PUMP | | **: SHIMADZU LC-20AD** |
| | COLUMN | | **: Kinetex C18 (2. 6 *µ*m, 3. 0×150 mm)** |
| | MOBILE PHASE | | **: A: 10mM HCOONH₄** |
| | | | **B: Acetonitrile** |
| | | | **0. 00→2. 00(min) A/B=40:60→10:90** |
| | | | **2. 00→3. 50(min) A/B=10:90-0:100** |
| | | | **3. 50→5. 50(min)A/B=0:100** |
| | | | **5. 50→5. 51(min) A/B=0:100→40:60** |
| | | | **5. 51→6. 50(min) A/B=40:60** |
| | COLUMN TEMPERATURE | | **: 50°C** |
| | FLOW RATE | | **: 0. 6 mL/min** |
| | INJECTION AMOUNT | | **: 0. 02mL**(DISSOLVED IN 0.1 mL of 70% ACETONITRILE) |

| | **Run Time** | | **: 6. 5 min** |
|---|---|---|---|
| MS/MS CONDITIONS | | | |
| | **MS/MS** | | **: API5000 (AB Sciex)** |
| | **IONIZATION** | | **: ESI (Positive)** |
| | | **CAD** | **: 5 psi** |
| | | **CUR** | **: 11 psi** |
| | | **Gas 1** | **: 40 psi** |
| | | **Gas 2** | **: 70 psi** |
| | | **IonSpray Voltage** | **: 5500 volts** |

| | | ION SOURCE TEMPERATURE | **: 600 °C** |
|---|---|---|---|
| **MEASURED ION (*m*/*z*)** | | | |
| | | **E1-TfpyHMP** | **: 525 → 308** |
| | | **E1-¹³C₄-TfpyHMP** | **: 529 → 312** |

### <<Statistical analysis>>

The measured estrone (E1) concentration and estradiol (E2) concentration in the serum were subjected to analysis of covariance (ANCOVA), and adjusted with the E1 concentration in the serum at the start of the test (before the first intake) or the E2 concentration in the serum at the start of the test (before the first intake), and age. As a difference between two groups, a p value (probability value) of p < 0.05 was considered as being statistically significant. The results are presented in Table 8 below.

**[Table 8]**

| GROUP | CONCENTRATION (pg/mL) | |
|---|---|---|
| | ESTRONE (E1) | ESTRADIOL (E2) |
| CONTROL FOOD PRODUCT INTA KE GROUP | 25.12 ± 1.08 | 13.18 ± 1.16 |
| TEST FOOD PRODUCT INTAKE GROUP | 31.62 ± 1.08* | 21.38 ± 1.17* |

| | | |
|---|---|---|
| * : p<0.05 | | |

It was found from the results of Table 8 that a significant increase in the estrogen (E1 and E2) concentration in blood was observed in the test food product intake group with intake of the food product including three kinds of lactic acid bacteria.

Embodiments of the present invention includes, for example, the following.
<1> A glucuronidase activator including an active ingredient that is a *Levilactobacillus brevis* strain I-3141 deposited under accession number CECT 7480.
<2> The glucuronidase activator according to <1>, in which the *Levilactobacillus brevis* strain I-3141 is a dried product or a disrupted product.
<3> The glucuronidase activator according to <1> or <2> further including a lactic acid bacterium assigned to *Lactoplantibacillus plantarum,* a lactic acid bacterium assigned to *Pediococcus acidilactici,* or both.
<4> The glucuronidase activator according to <3>, in which the lactic acid bacterium assigned to *Lactoplantibacillus plantarum* is a *Lactoplantibacillus plantarum* strain F-2096 deposited under accession number CECT 7481.
<5> The glucuronidase activator according to <3> or <4>, in which the lactic acid bacterium assigned to *Pediococcus acidilactici* is a *Pediococcus acidilactici* strain F-1033 deposited under accession number CECT 7483.
<6> The glucuronidase activator according to any one of <1> to <5> that is used for increasing an estrogen level in a body.
<7> The glucuronidase activator according to any one of <1> to <5> that is used for improving estrogen metabolism.
<8> The glucuronidase activator according to any one of <1> to <5> that is used for improving climacteric symptoms.
<9> A method for enhancing glucuronidase activity, including administering of a *Levilactobacillus brevis* strain I-3141 deposited under accession number CECT 7480 to a subject to enhance the glucuronidase activity.
<10> The method for enhancing the glucuronidase activity according to <9>, in which the *Levilactobacillus brevis* strain I-3141 is a dried product or a disrupted product.
<11> The method for enhancing the glucuronidase activity according to <9> or <10>, further using a lactic acid bacterium assigned to *Lactoplantibacillus plantarum,* a lactic acid bacterium assigned to *Pediococcus acidilactici,* or both.
<12> The method for enhancing the glucuronidase activity according to <11>, in which the lactic acid bacterium assigned to the *Lactoplantibacillus plantarum* is a *Lactoplantibacillus plantarum* strain F-2096 deposited under accession number CECT 7481.
<13> The method for enhancing the glucuronidase activity according to <11> or <12>, in which the lactic acid bacterium assigned to the *Pediococcus acidilactici* is a *Pediococcus acidilactici* strain F-1033 deposited under accession number CECT 7483.
<14> A pharmaceutical composition including the glucuronidase activator according to any one of <1> to <8> and a pharmaceutically acceptable carrier.
<15> The pharmaceutical composition according to <14> that is an oral composition.
<16> An edible composition including the glucuronidase activator according to any one of <1> to <8> and an edible ingredient.
<17> The edible composition according to <16> that is a probiotic composition.
<18> The edible composition according to <16> or <17> packaged in a container, the container having a label or an instruction indicating, as a function of the edible composition, at least one selected from the group consisting of:
   (a) increase in an estrogen level in a body;
   (b) improvement of estrogen metabolism; and
   (c) improvement of climacteric symptoms.
<19> The edible composition according to <18>, wherein the edible composition is a health functional food.
<20> An oral composition including a *Levilactobacillus brevis* strain I-3141 deposited under accession number CECT 7480, *Lactoplantibacillus plantarum* deposited under accession number CECT 7481, and *Pediococcus acidilactici* deposited under accession number CECT 7483.
<21> A microorganism-containing composition including a *Levilactobacillus brevis* strain I-3141 deposited under accession number CECT 7480.
<22> The microorganism-containing composition according to <21>, further including a lactic acid bacterium assigned to *Lactoplantibacillus plantarum,* a lactic acid bacterium assigned to *Pediococcus acidilactici,* or both.
<23> The microorganism-containing composition according to <22>, in which the lactic acid bacterium assigned to the *Lactoplantibacillus plantarum* is a *Lactoplantibacillus plantarum* strain F-2096 deposited under accession number CECT 7481.
<24> The microorganism-containing composition according to <22> or <23>, in which the lactic acid bacterium assigned to *Pediococcus acidilactici* is a *Pediococcus acidilactici* strain F-1033 deposited under accession number CECT 7483.
<25> Use of a *Levilactobacillus brevis* strain I-3141 deposited under accession number CECT 7480 for enhancing glucuronidase activity.
<26> Use of a *Levilactobacillus brevis* strain I-3141 deposited under accession number CECT 7480 for increasing an estrogen level.
<27> Use of a *Levilactobacillus brevis* strain I-3141 deposited under accession number CECT 7480 for improving estrogen metabolism.
<28> Use of the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24>, for increasing an estrogen level.
<29> Use of the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24>, for improving estrogen metabolism.
<30> Use of the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24>, for improving climacteric symptoms.
<31> A microorganism that is a *Levilactobacillus brevis* strain I-3141 deposited under accession number CECT 7480 and has glucuronidase activity.
<32> A method of enhancing glucuronidase activity including orally administering the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24> to a subject.
<33> A method of increasing an estrogen level in a body including orally administering the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24> to a subject.
<34> A method of improving estrogen metabolism including orally administering the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24> to a subject.
<35> A method of improving climacteric symptoms including orally administering the glucuronidase activator according to any one of <1> to <8>, the pharmaceutical composition according to <14> or <15>, the edible composition according to any one of <16> to <19>, the oral composition according to <20>, or the microorganism-containing composition according to any one of <21> to <24> to a subject.

This international application claims priority based on Japanese Patent Application No. 2022-042917 filed on March 17, 2022, the entire contents of which are incorporated herein by reference.

### [Accession Number]

CECT 7480
CECT 7481
CECT 7483

### [Sequence Listing]

B220212.xml

## Claims

1. A glucuronidase activator comprising:
an active ingredient that is a *Levilactobacillus brevis* strain I-3141 deposited under accession number CECT 7480.

2. The glucuronidase activator according to claim 1, further comprising:
a lactic acid bacterium assigned to *Lactoplantibacillus plantarum,* a lactic acid bacterium assigned to *Pediococcus acidilactici,* or both.

3. The glucuronidase activator according to claim 2,
wherein the lactic acid bacterium assigned to *Lactoplantibacillus plantarum* is a *Lactoplantibacillus plantarum* strain F-2096 deposited under accession number CECT 7481.

4. The glucuronidase activator according to claim 2 or 3,
wherein the lactic acid bacterium assigned to *Pediococcus acidilactici* is a *Pediococcus acidilactici* strain F-1033 deposited under accession number CECT 7483.

5. The glucuronidase activator according to any one of claims 1 to 4,
wherein the glucuronidase activator is used for increasing an estrogen level in a body.

6. The glucuronidase activator according to any one of claims 1 to 4,
wherein the glucuronidase activator is used for improving estrogen metabolism.

7. The glucuronidase activator according to any one of claims 1 to 4,
wherein the glucuronidase activator is used for improving climacteric symptoms.

8. A method of enhancing glucuronidase activity, the method comprising:
administering a *Levilactobacillus brevis* strain I-3141 deposited under accession number CECT 7480 to a subject to enhance the glucuronidase activity.

9. A pharmaceutical composition comprising:
the glucuronidase activator according to any one of claims 1 to 7; and
a pharmaceutically acceptable carrier.

10. The pharmaceutical composition according to claim 9,
wherein the pharmaceutical composition is an oral composition.

11. An edible composition comprising:
the glucuronidase activator according to any one of claims 1 to 7; and
an edible ingredient.

12. The edible composition according to claim 11,
wherein the edible composition is a probiotic composition.

13. The edible composition according to claim 11 or 12,
wherein the edible composition is packaged in a container, and the container has a label or an instruction indicating, as a function of the edible composition, at least one selected from the group consisting of:
(a) increase of an estrogen level in a body;
(b) improvement of estrogen metabolism; and
(c) improvement of climacteric symptoms.

14. An oral composition comprising:
*a Levilactobacillus brevis* strain I-3141 deposited under accession number CECT 7480;
*Lactoplantibacillus plantarum* deposited under accession number CECT 7481; and
*Pediococcus acidilactici* deposited under accession number CECT 7483.

15. A method of increasing an estrogen level, the method comprising:
orally administering the glucuronidase activator according to any one of claims 1 to 4, the pharmaceutical composition according to claim 9 or 10, the edible composition according to any one of claims 11 to 13, or the oral composition according to claim 14 to a subject.

16. A method of improving estrogen metabolism, the method comprising:
orally administering the glucuronidase activator according to any one of claims 1 to 4, the pharmaceutical composition according to claim 9 or 10, the edible composition according to any one of claims 11 to 13, or the oral composition according to claim 14 to a subject.

17. A method of improving climacteric symptoms, the method comprising:
orally administering the glucuronidase activator according to any one of claims 1 to 4, the pharmaceutical composition according to claim 9 or 10, the edible composition according to any one of claims 11 to 13, or the oral composition according to claim 14 to a subject.
